# EUROPEAN PATENT SPECIFICATION

(11) **EP 1 603 521 B1**
(45) Date of publication and mention of the grant of the patent: **17.06.2020**
(21) Application number: 04757640.0
(22) Date of filing: 18.03.2004
(51) Int. Cl.: A01N 43/40, A01P 3/00, A01N 59/16, A61K 8/27

(54) **AUGMENTATION OF PYRITHIONE ACTIVITY OR A POLYVALENT METAL SALT OF PYRITHIONE ACTIVITY BY ZINC-CONTAINING LAYERED MATERIAL**
VERSTÄRKUNG DER AKTIVITÄT VON PYRITHION ODER EINES POLYVALENTEN METALLSALZES VON PYRITHION DURCH EIN ZINKHALTIGES SCHICHTMATERIAL
AUGMENTATION DE L'ACTIVITE DE LA PYRITHIONE OU DE L'ACTIVITE D'UN SEL METALLIQUE POLYVALENT DE LA PYRITHIONE PAR UN MATERIAU EN COUCHES CONTENANT DU ZINC

(30) Priority: 18.03.2003 US 455780 P
(43) Date of publication of application: 14.12.2005
(73) Proprietor: THE PROCTER & GAMBLE COMPANY, Cincinnati, OH 45202 (US); Arch Chemicals, Inc., Cheshire, CT 06410 (US)
(72) Inventor: SCHWARTZ, James, Robert, West Chester, OH 45069 (US); JOHNSON, Eric, Scott, Hamilton, OH 45011 (US); KING, Bonnie, Theresa, Alexandria, KY 41001 (US); AKRED, Joyce, Ross, Cincinnati, OH 45241 (US); POLSON, George, Harwinton, CT 06791 (US); TURLEY, Patricia, A., Orange, CT 06477 (US)
(74) Representative: P&G Patent Germany
(86) International application number: PCT/US2004/008485
(87) International publication number: WO 2004/082649

(56) References cited:
- WO-A-98/47372
- WO-A1-01/00151
- WO-A1-98/36904
- FR-A- 2 593 801
- US-A- 5 227 156
- DATABASE EMBASE [Online] ELSEVIER SCIENCE PUBLISHERS, AMSTERDAM, NL; 1990, VAN CUTSEM J ET AL: "The in vitro antifungal activity of ketoconazole, zinc pyrithione, and selenium sulfide against Pityrosporum and their efficacy as a shampoo in the treatment of experimental pityrosporosis in guinea pigs" XP002288119 Database accession no. EMB-1990186475 & JOURNAL OF THE AMERICAN ACADEMY OF DERMATOLOGY 1990 UNITED STATES, vol. 22, no. 6 I, 1990, pages 993-998, ISSN: 0190-9622
- DATABASE WPI Section Ch, Week 200376 Derwent Publications Ltd., London, GB; Class B06, AN 2003-812501 XP002288045 & WO 03/082229 A1 (SHISEIDO CO LTD) 9 October 2003 (2003-10-09) & WO 03/082229 A (YOKOYAMA HIROYUKI ; KAWAI ERIKO (JP); OGAWA SHIGEYUKI (JP); SAKUMA KEN) 9 October 2003 (2003-10-09)

## Description

### Field

The present invention relates to a composition comprising an effective amount of metal salts of pyrithione and an effective amount of a zinc containing layered material which provides an augmentation factor greater than 1. More particularly, the present invention relates to personal care compositions and methods of treating microbial and fungal infections on the skin or scalp. Even more particularly, the present invention relates to methods for the treatment of dandruff and compositions, which provide improved anti-dandruff activity.

### Background

Various anti-dandruff compositions are commercially available or otherwise known in the shampoo art. These compositions typically comprise detersive surfactants and particulate, crystalline anti-microbial agents dispersed and suspended throughout the composition. Anti-microbial agents used for this purpose include sulfur, selenium sulfide and polyvalent metal salts of pyridinethione. During the shampooing process, these anti-microbial agents deposit on the scalp to provide anti-dandruff activity. Soluble anti-dandruff agents, such as ketoconazole and octopirox, are also known in the art.

Polyvalent metal salts of pyrithione (also known as 1-hydroxy-2-pyridinethione; 2-pyridinethiol-1-oxide; 2-pyridinethione; 2-mercaptopyridine-N-oxide; pyridinethione; and pyridinethione-N-oxide) are known to be effective biocidal agents and are widely used as fungicides and bacteriocides in paints and metalworking fluids. Polyvalent metal salts of pyrithione are also used as fungicides and bacteriocides in personal care compositions such as foot powders and anti-dandruff shampoos. The polyvalent metal salts of pyrithione are only sparingly soluble in water and include magnesium pyrithione, barium pyrithione, bismuth pyrithione, strontium pyrithione, copper pyrithione, zinc pyrithione, cadmium pyrithione, and zirconium pyrithione.

Zinc and copper pyrithione are especially useful as anti-microbial agents in personal care compositions. Zinc pyrithione is known as an anti-dandruff component in shampoos. While pyrithione biocides have proven useful for a wide range of applications, the utility of these compounds is limited to the control of select species and strains of fungi and bacteria. Further, while higher concentrations of pyrithione salts have been observed to control the growth of a wider range of organisms, the useful amount of polyvalent metal salts of pyrithione that can be added to a commercial product is limited by efficacy and economic considerations, and environmental concerns.

International patent application WO98/47372 A1 is related to a personal care composition comprising: (a) water or an alcohol, (b) at least one dispersant or surfactant, and (c) as an antimicrobial or preservative additive, particles of an in-situ trans chelation reaction product of a zinc compound selected from the group consisting of zinc salts of organic acids, zinc salts of inorganic acids, zinc hydroxide, zinc oxide, and combinations thereof, said zinc compound being soluble in said water or alcohol, with pyrithione acid or a pyrithione salt that is soluble in said water or alcohol.

US Patent US 5,227,156 refers to the activity of a thiazolinone preservative, in an antidandruff shampoo containing pyrithione, which is maintained by adding a stabilizer comprising a zinc compound.

International patent application WO98/36904 A1 is about an aqueous antimicrobial coating composition, characterized by antimildew efficacy and protected against discoloration attributable to the presence of pyrithione therein, said composition being selected from the group consisting of water-based paints, adhesives, caulks and sealants, and combinations thereof, said composition comprising water, a pyrithione salt or acid, an organic base medium and a zinc compound, said zinc ion being present in said composition in an amount of from 0.001 % to 10 % based upon the weight of the coating composition.

International patent application WO01/00151 A1 sets out topical compositions for the treatment of microbial infections on the skin or scalp which include a polyvalent metal salt of pyrithione and include a metal ion source.

Despite the options available, consumers still desire a shampoo that provides superior anti-dandruff efficacy versus currently marketed products; as such consumers have found that dandruff is still prevalent. Such a superior efficacy can be difficult to achieve.

### Summary

An embodiment of the present invention is directed to a composition comprising: an effective amount of zinc pyrithione comprised from 0.01% to 5%, based upon the total weight of the composition; an effective amount of a zinc-containing layered material comprised from 0.001% to 10%, based upon the total weight of the composition. The zinc-containing layered material is selected from the group consisting of basic zinc carbonate, zinc carbonate hydroxide, hydrozincite, zinc copper carbonate hydroxide, aurichalcite, copper zinc carbonate hydroxide, rosasite, phyllosilicate containing zinc ions, layered double hydroxide, hydroxy double salts and mixtures thereof. Preferably, the zinc-containing layered material may be selected from the group consisting of zinc carbonate hydroxide, hydrozincite, basic zinc carbonate and mixtures thereof. More preferably, the zinc-containing layered material may be hydrozincite or basic zinc carbonate. Most preferably, the zinc containing layered material may be basic zinc carbonate.

The composition as set out hereinbefore may be used for treating microbial infections.

The composition as set out hereinbefore may be used for treating fungal infections.

The composition as set out hereinbefore may be used for treating dandruff.

These and other features, aspects, and advantages of the present invention will become evident to those skilled in the art from a reading of the present disclosure.

### Detailed Description

While the specification concludes with claims, which particularly point out and distinctly claim the invention, it is believed the present invention will be better understood from the following description.

Anti-microbial compounds possess an intrinsic capability to control microbial populations. It is well known that secondary materials can modulate that activity, often decreasing it, but sometimes increasing it. The latter effect is desirable in that it has the benefit of either increasing overall anti-microbial effectiveness or maintaining activity at lower levels of the anti-microbial material (i.e., increased efficiency). These are both desirable outcomes as increasing anti-microbial efficiency (the amount required to achieve efficacy) improves both the cost and safety of using such materials.

Materials which have little or no independent activity, yet increase the activity of an anti-microbial material, are "augmenting" agents. Augmenting agents are generally specific to the material being augmented, forming a unique combination and often because there is a specific chemical or biological mechanism required to achieve the augmentation effect. As such, it is quite rare to identify augmenting agents because, frequently, the underlying anti-microbial mechanisms are not well known even for the primary anti-microbial material.

This is the case for zinc pyrithione (ZPT), a well-known anti-microbial material lacking a well-defined mechanism of action. Applicants have surprisingly discovered a family of materials that provide this augmentation activity. These materials are zinc-containing layered materials (ZLM's) in which zinc is present in either the primary layers or intercalated between the layers. Such materials reduce the amount of ZPT required to achieve total growth inhibition of microbial populations. Thus, ZLM's have been discovered as augmenting agents of ZPT.

The present invention can comprise, consist of, or consist essentially of the essential elements and limitations of the invention described herein, as well any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

The components and/or steps, including those, which may optionally be added, of the various embodiments of the present invention, are described in detail below.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more"

Herein, "comprising" means that other steps and other ingredients, which do not affect the end result, can be added. This term encompasses the terms "consisting of' and "consisting essentially of". The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.

### A. Zinc-Containing Layered Material

The composition of the present invention includes an effective amount of a zinc-containing layered material comprised from 0.001 % to 10% based upon the total weight of the composition; preferably from 0.01% to 7%; more preferably from 0.1% to 5%.

The zinc-containing layered material is selected from the group consisting of basic zinc carbonate, zinc carbonate hydroxide, hydrozincite, zinc copper carbonate hydroxide, aurichalcite, copper zinc carbonate hydroxide, rosasite, phyllosilicate containing zinc ions, layered double hydroxide, hydroxy double salts and mixtures thereof, preferably wherein the zinc-containing layered material is selected from the group consisting of zinc carbonate hydroxide, hydrozincite, basic zinc carbonate and mixtures thereof, more preferably wherein the zinc-containing layered material is hydrozincite or basic zinc carbonate, most preferably wherein the zinc containing layered material is basic zinc carbonate.

Zinc-containing layered structures are those with crystal growth primarily occurring in two dimensions. It is conventional to describe layer structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLM's) may have zinc incorporated in the layers and/or be components of the gallery ions.

Many ZLM's occur naturally as minerals. Common examples include hydrozincite (zinc carbonate hydroxide), basic zinc carbonate, aurichalcite (zinc copper carbonate hydroxide), rosasite (copper zinc carbonate hydroxide) and many related minerals that are zinc-containing. Natural ZLM's can also occur wherein anionic layer species such as clay-type minerals (e.g., phyllosilicates) contain ion-exchanged zinc gallery ions. All of these natural materials can also be obtained synthetically or formed in situ in a composition or during a production process.

Another common class of ZLM's, which are often, but not always, synthetic, is layered doubly hydroxides, which are generally represented by the formula [M²⁺₁₋ₓM³⁺ₓ(OH)₂]^{x+} A^{m-}_{x/m}·nH₂O and some or all of the divalent ions (M²⁺) would be represented as zinc ions (Crepaldi, EL, Pava, PC, Tronto, J, Valim, JB J. Colloid Interfac. Sci. 2002, 248, 429-42).

Yet another class of ZLM's can be prepared called hydroxy double salts (Morioka, H., Tagaya, H., Karasu, M, Kadokawa, J, Chiba, K Inorg. Chem. 1999, 38, 4211-6). Hydroxy double salts can be represented by the general formula [M²⁺₁₋ₓM²⁺₁₊ₓ(OH)_{3(1-y)}]⁺ Aⁿ⁻_{(1=3y)/n}·nH₂O where the two metal ion may be different; if they are the same and represented by zinc, the formula simplifies to [Zn₁₊ₓ(OH)₂]^{2x+} 2x A⁻·nH₂O. This latter formula represents (where x=0.4) common materials such as zinc hydroxychloride and zinc hydroxynitrate. These are related to hydrozincite as well wherein a divalent anion replaces the monovalent anion. These materials can also be formed in situ in a composition or in or during a production process.

Commercially available sources of basic zinc carbonate include Zinc Carbonate Basic (Cater Chemicals: Bensenville, IL, USA), Zinc Carbonate (Shepherd Chemicals: Norwood, OH, USA), Zinc Carbonate (CPS Union Corp.: New York, NY, USA), Zinc Carbonate (Elementis Pigments: Durham, UK), and Zinc Carbonate AC (Bruggemann Chemical: Newtown Square, PA, USA).

Basic zinc carbonate, which also may be referred to commercially as "Zinc Carbonate" or "Zinc Carbonate Basic" or "Zinc Hydroxy Carbonate", is a synthetic version consisting of materials similar to naturally occurring hydrozincite. The idealized stoichiometry is represented by Zn₅(OH)₆(CO₃)₂ but the actual stoichiometric ratios can vary slightly and other impurities may be incorporated in the crystal lattice.

### B. Pyrithione or a Polyvalent metal salt of Pyrithione

The present composition comprises zinc salt of 1-hydroxy-2-pyridinethione (known as "zinc pyrithione" or "ZPT"); more preferably ZPT in platelet particle form, wherein the particles have an average size of up to 20µm, preferably up to 5µm, more preferably up to 2.5µm.

Pyridinethione anti-microbial and anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

It is further contemplated that when ZPT is used as the anti-microbial particulate in the anti-microbial compositions herein, that an additional benefit of hair growth or re-growth may be stimulated or regulated, or both, or that hair loss may be reduced or inhibited, or that hair may appear thicker or fuller.

Zinc pyrithione may be made by reacting 1-hydroxy-2-pyridinethione (i.e., pyrithione acid) or a soluble salt thereof with a zinc salt (e.g. zinc sulfate) to form a zinc pyrithione precipitate, as illustrated in U.S. Patent No. 2,809,971.

The composition include an effective amount of zinc pyrithione comprised from 0.01% to 5% based upon the total weight of the composition; preferably from 0.1% to 2%.

In embodiments having a zinc-containing layered material and a pyrithione, the ratio of zinc-containing layered material to pyrithione is preferably from 5:100 to 10:1; more preferably from 2:10 to 5:1; more preferably still from 1:2 to 3:1.

### C. Topical Carrier

In a preferred embodiment, the composition of the present invention is in the form of a topical composition, which includes a topical carrier. Preferably, the topical carrier is selected from a broad range of traditional personal care carriers depending on the type of composition to be formed. By suitable selections of compatible carriers, it is contemplated that such a composition is prepared in the form of daily skin or hair products including conditioning treatments, cleansing products, such as hair and/or scalp shampoos, body washes, hand cleansers, water-less hand sanitizer/cleansers, facial cleansers and the like.

In a preferred embodiment, the carrier is water. Preferably the compositions of the present invention comprise from 40% to 95% water by weight of the composition; preferably from 50% to 85%, more preferably still from 60% to 80%; based upon the total weight of the composition.

### D. Detersive Surfactant

The composition of the present invention includes a detersive surfactant. The detersive surfactant component is included to provide cleaning performance to the composition. The detersive surfactant component in turn comprises anionic detersive surfactant, zwitterionic or amphoteric detersive surfactant, or a combination thereof. Such surfactants should be physically and chemically compatible with the essential components described herein, or should not otherwise unduly impair product stability, aesthetics or performance.

Suitable anionic detersive surfactant components for use in the composition herein include those which are known for use in hair care or other personal care cleansing compositions. The concentration of the anionic surfactant component in the composition should be sufficient to provide the desired cleaning and lather performance, and generally range from 5% to 50%, preferably from 8% to 30%, more preferably from 10% to 25%, even more preferably from 12% to 22%; based upon the total weight of the composition.

Preferred anionic surfactants suitable for use in the compositions are the alkyl and alkyl ether sulfates. These materials have the respective formulae ROSO₃M and RO(C₂H₄O)ₓSO₃M, wherein R is alkyl or alkenyl of from 8 to 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium.

Preferably, R has from 8 to 18 carbon atoms, more preferably from 10 to 16 carbon atoms, even more preferably from 12 to 14 carbon atoms, in both the alkyl and alkyl ether sulfates. The alkyl ether sulfates are typically made as condensation products of ethylene oxide and monohydric alcohols having from 8 to 24 carbon atoms. The alcohols can be synthetic or they can be derived from fats, e.g., coconut oil, palm kernel oil, tallow. Lauryl alcohol and straight chain alcohols derived from coconut oil or palm kernel oil are preferred. Such alcohols are reacted with between 0 and 10, preferably from 2 to 5, more preferably 3, molar proportions of ethylene oxide, and the resulting mixture of molecular species having, for example, an average of 3 moles of ethylene oxide per mole of alcohol, is sulfated and neutralized.

Other suitable anionic detersive surfactants are the water-soluble salts of organic, sulfuric acid reaction products conforming to the formula [R¹-SO₃-M] where R¹ is a straight or branched chain, saturated, aliphatic hydrocarbon radical having from 8 to 24, preferably 10 to 18, carbon atoms; and M is a cation described hereinbefore.

Still other suitable anionic detersive surfactants are the reaction products of fatty acids esterified with isethionic acid and neutralized with sodium hydroxide where, for example, the fatty acids are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Pat. Nos. 2,486,921; 2,486,922; and 2,396,278.

Other anionic detersive surfactants suitable for use in the compositions are the succinnates, examples of which include disodium N-octadecylsulfosuccinnate; disodium lauryl sulfosuccinate; diammonium lauryl; tetrasodium N-(1,2-dicarboxyethyl)-N-octadecylsulfosuccinnate; diamyl ester of sodium sulfosuccinic acid; dihexyl ester of sodium sulfosuccinic acid; and dioctyl esters of sodium sulfosuccinic acid.

Other suitable anionic detersive surfactants include olefin sulfonates having 10 to 24 carbon atoms. In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process. An example of such an alpha-olefin sulfonate mixture is described in U.S. Patent 3,332,880.

Another class of anionic detersive surfactants suitable for use in the compositions are the beta-alkyloxy alkane sulfonates. These surfactants conform to the formula 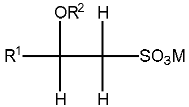 where R¹ is a straight chain alkyl group having from 6 to 20 carbon atoms, R² is a lower alkyl group having from 1 to 3 carbon atoms, preferably 1 carbon atom, and M is a water-soluble cation as described hereinbefore.

Preferred anionic detersive surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof.

Suitable amphoteric or zwitterionic detersive surfactants for use in the composition herein include those, which are known for use in hair care or other personal care cleansing. Concentration of such amphoteric detersive surfactants preferably ranges from 0.5% to 20%, preferably from 1% to 10%. Examples of suitable zwitterionic or amphoteric surfactants are described in U.S. Pat. Nos. 5,104,646 (Bolich Jr. et al.), 5,106,609 (Bolich Jr. et al.).

Amphoteric detersive surfactants suitable for use in the composition are well known in the art, and include those surfactants broadly described as derivatives of aliphatic secondary and tertiary amines in which the aliphatic radical can be straight or branched chain and wherein one of the aliphatic substituents contains from 8 to 18 carbon atoms and one contains an anionic group such as carboxy, sulfonate, sulfate, phosphate, or phosphonate. Preferred amphoteric detersive surfactants for use in the present invention include cocoamphoacetate, cocoamphodiacetate, lauroamphoacetate, lauroamphodiacetate, and mixtures thereof.

Zwitterionic detersive surfactants suitable for use in the composition are well known in the art, and include those surfactants broadly described as derivatives of aliphatic quaternaryammonium, phosphonium, and sulfonium compounds, in which the aliphatic radicals can be straight or branched chain, and wherein one of the aliphatic substituents contains from 8 to 18 carbon atoms and one contains an anionic group such as carboxy, sulfonate, sulfate, phosphate or phosphonate. Zwitterionics such as betaines are preferred.

The compositions of the present invention may further comprise additional surfactants for use in combination with the anionic detersive surfactant component described hereinbefore. Suitable optional surfactants include nonionic and cationic surfactants. Any such surfactant known in the art for use in hair or personal care products may be used, provided that the optional additional surfactant is also chemically and physically compatible with the essential components of the composition, or does not otherwise unduly impair product performance, aesthetics or stability. The concentration of the optional additional surfactants in the composition may vary with the cleansing or lather performance desired, the optional surfactant selected, the desired product concentration, the presence of other components in the composition, and other factors well known in the art.

Examples of other anionic, zwitterionic, amphoteric or optional additional surfactants suitable for use in the compositions are described in McCutcheon's, Emulsifiers and Detergents, 1989 Annual, published by M. C. Publishing Co., and U.S. Pat. Nos. 3,929,678, 2,658,072; 2,438,091; 2,528,378.

### E. Dispersed Particles

The composition of the present invention may include dispersed particles. In the compositions of the present invention, it is preferable to incorporate at least 0.025% by weight of the dispersed particles, more preferably at least 0.05%, still more preferably at least 0.1%, even more preferably at least 0.25%, and yet more preferably at least 0.5% by weight of the dispersed particles. In the compositions of the present invention, it is preferable to incorporate no more than 20% by weight of the dispersed particles, more preferably no more than 10%, still more preferably no more than 5%, even more preferably no more than 3%, and yet more preferably no more than 2% by weight of the dispersed particles.

### F. Aqueous Carrier

The compositions of the present invention are typically in the form of pourable liquids (under ambient conditions). The compositions will therefore typically comprise an aqueous carrier, which is present at a level of from 20% to 95%, preferably from 60% to 85%. The aqueous carrier may comprise water, or a miscible mixture of water and organic solvent, but preferably comprises water with minimal or no significant concentrations of organic solvent, except as otherwise incidentally incorporated into the composition as minor ingredients of other essential or optional components.

### G. Additional Components

The compositions of the present invention may further comprise one or more optional components known for use in hair care or personal care products, provided that the optional components are physically and chemically compatible with the essential components described herein, or do not otherwise unduly impair product stability, aesthetics or performance. Individual concentrations of such optional components may range from 0.001% to 10%.

Examples of optional components for use in the composition include cationic polymers, conditioning agents (hydrocarbon oils, fatty esters, silicones), anti dandruff agents, suspending agents, viscosity modifiers, dyes, nonvolatile solvents or diluents (water soluble and insoluble), pearlescent aids, foam boosters, additional surfactants or nonionic cosurfactants, pediculocides, pH adjusting agents, perfumes, preservatives, chelants, proteins, skin active agents, sunscreens, UV absorbers, and vitamins, minerals, herbal/fruit/food extracts, sphingolipids derivatives or synthetical derivative, and clay.

### 1. Cationic Polymers

The compositions of the present invention may contain a cationic polymer. Concentrations of the cationic polymer in the composition typically range from 0.05% to 3%, preferably from 0.075% to 2.0%, more preferably from 0.1% to 1.0%. Preferred cationic polymers will have cationic charge densities of at least 0.9 meq/gm, preferably at least 1.2 meq/gm, more preferably at least 1.5 meq/gm, but also preferably less than 7 meq/gm, more preferably less than 5 meq/gm. Herein, "cationic charge density" of a polymer refers to the ratio of the number of positive charges on the polymer to the molecular weight of the polymer. The average molecular weight of such suitable cationic polymers will generally be between 10,000 and 10 million, preferably between 50,000 and 5 million, more preferably between 100,000 and 3 million.

Suitable cationic polymers for use in the compositions of the present invention contain cationic nitrogen-containing moieties such as quaternary ammonium or cationic protonated amino moieties. The cationic protonated amines can be primary, secondary, or tertiary amines (preferably secondary or tertiary), depending upon the particular species and the selected pH of the composition. Any anionic counterions can be used in association with the cationic polymers so long as the polymers remain soluble in water, in the composition, or in a coacervate phase of the composition, and so long as the counterions are physically and chemically compatible with the essential components of the composition or do not otherwise unduly impair product performance, stability or aesthetics. Examples of such counterions include halides (e.g., chloride, fluoride, bromide, iodide), sulfate and methylsulfate.

Examples of such polymers are described in the CTFA Cosmetic Ingredient Dictionary, 3rd edition, edited by Estrin, Crosley, and Haynes, (The Cosmetic, Toiletry, and Fragrance Association, Inc., Washington, D.C. (1982)).

Examples of suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

Suitable cationic protonated amino and quaternary ammonium monomers, for inclusion in the cationic polymers of the composition herein, include vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts.

Other suitable cationic polymers for use in the compositions include copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16); copolymers of 1-vinyl-2-pyrrolidone and dimethylaminoethyl methacrylate (referred to in the industry by CTFA as Polyquaternium-11); cationic diallyl quaternary ammonium-containing polymers, including, for example, dimethyldiallylammonium chloride homopolymer, copolymers of acrylamide and dimethyldiallylammonium chloride (referred to in the industry by CTFA as Polyquaternium 6 and Polyquaternium 7, respectively); amphoteric copolymers of acrylic acid including copolymers of acrylic acid and dimethyldiallylammonium chloride (referred to in the industry by CTFA as Polyquaternium 22), terpolymers of acrylic acid with dimethyldiallylammonium chloride and acrylamide (referred to in the industry by CTFA as Polyquaternium 39), and terpolymers of acrylic acid with methacrylamidopropyl trimethylammonium chloride and methylacrylate (referred to in the industry by CTFA as Polyquaternium 47). Preferred cationic substituted monomers are the cationic substituted dialkylaminoalkyl acrylamides, dialkylaminoalkyl methacrylamides, and combinations thereof. These preferred monomers conform to the formula 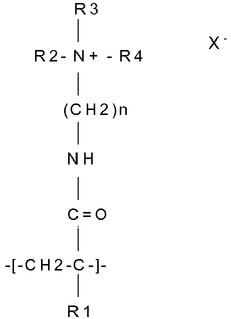 wherein R¹ is hydrogen, methyl or ethyl; each of R², R³ and R⁴ are independently hydrogen or a short chain alkyl having from 1 to 8 carbon atoms, preferably from 1 to 5 carbon atoms, more preferably from 1 to 2 carbon atoms; n is an integer having a value of from 1 to 8, preferably from 1 to 4; and X is a counterion. The nitrogen attached to R², R³ and R⁴ may be a protonated amine (primary, secondary or tertiary), but is preferably a quaternary ammonium wherein each of R², R³ and R⁴ are alkyl groups am example of which is polymethyacrylamidopropyl trimonium chloride, available under the trade name Polycare 133, from Rhone-Poulenc, Cranberry, N.J., U.S.A.

Other suitable cationic polymers for use in the composition include polysaccharide polymers, such as cationic cellulose derivatives and cationic starch derivatives. Suitable cationic polysaccharide polymers include those, which conform to the formula 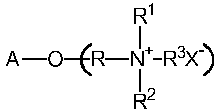 wherein A is an anhydroglucose residual group, such as a starch or cellulose anhydroglucose residual; R is an alkylene oxyalkylene, polyoxyalkylene, or hydroxyalkylene group, or combination thereof; R1, R2, and R3 independently are alkyl, aryl, alkylaryl, arylalkyl, alkoxyalkyl, or alkoxyaryl groups, each group containing up to 18 carbon atoms, and the total number of carbon atoms for each cationic moiety (i.e., the sum of carbon atoms in R1, R2 and R3) preferably being 20 or less; and X is an anionic counterion as described in hereinbefore.

Preferred cationic cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA) in their Polymer LR, JR, and KG series of polymers. Other suitable types of cationic cellulose includes the polymeric quaternary ammonium salts of hydroxyethyl cellulose reacted with lauryl dimethyl ammonium-substituted epoxide referred to in the industry (CTFA) as Polyquaternium 24. These materials are available from Amerchol Corp. under the tradename Polymer LM-200.

Other suitable cationic polymers include cationic guar gum derivatives, such as guar hydroxypropyltrimonium chloride, specific examples of which include the Jaguar series commercially available from Rhone-Poulenc Incorporated and the N-Hance series commercially available from Aqualon Division of Hercules, Inc. Other suitable cationic polymers include quaternary nitrogen-containing cellulose ethers, some examples of which are described in U.S. Pat. No. 3,962,418. Other suitable cationic polymers include copolymers of etherified cellulose, guar and starch, some examples of which are described in U.S. Pat. No. 3,958,581. When used, the cationic polymers herein are either soluble in the composition or are soluble in a complex coacervate phase in the composition formed by the cationic polymer and the anionic, amphoteric and/or zwitterionic detersive surfactant component described hereinbefore. Complex coacervates of the cationic polymer can also be formed with other charged materials in the composition.

Techniques for analysis of formation of complex coacervates are known in the art. For example, microscopic analyses of the compositions, at any chosen stage of dilution, can be utilized to identify whether a coacervate phase has formed. Such coacervate phase will be identifiable as an additional emulsified phase in the composition. The use of dyes can aid in distinguishing the coacervate phase from other insoluble phases dispersed in the composition.

### 2. Nonionic polymers

Polyalkylene glycols having a molecular weight of more than 1000 are useful herein. Useful are those having the following general formula: 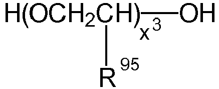 wherein R⁹⁵ is selected from the group consisting of H, methyl, and mixtures thereof. Polyethylene glycol polymers useful herein are PEG-2M (also known as Polyox WSR® N-10, which is available from Union Carbide and as PEG-2,000); PEG-5M (also known as Polyox WSR® N-35 and Polyox WSR® N-80, available from Union Carbide and as PEG-5,000 and Polyethylene Glycol 300,000); PEG-7M (also known as Polyox WSR® N-750 available from Union Carbide); PEG-9M (also known as Polyox WSR® N-3333 available from Union Carbide); and PEG-14 M (also known as Polyox WSR® N-3000 available from Union Carbide).

### 3. Conditioning agents

Conditioning agents include any material, which is used to give a particular conditioning benefit to hair and/or skin. In hair treatment compositions, suitable conditioning agents are those which deliver one or more benefits relating to shine, softness, combability, antistatic properties, wet-handling, damage, manageability, body, and greasiness. The conditioning agents useful in the compositions of the present invention typically comprise a water insoluble, water dispersible, non-volatile, liquid that forms emulsified, liquid particles. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein. Such conditioning agents should be physically and chemically compatible with the essential components of the composition, and should not otherwise unduly impair product stability, aesthetics or performance.

The concentration of the conditioning agent in the composition should be sufficient to provide the desired conditioning benefits, and as will be apparent to one of ordinary skill in the art. Such concentration can vary with the conditioning agent, the conditioning performance desired, the average size of the conditioning agent particles, the type and concentration of other components, and other like factors.

### 1. Silicones

The conditioning agent of the compositions of the present invention is preferably an insoluble silicone conditioning agent. The silicone conditioning agent particles may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile silicone materials ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

The concentration of the silicone conditioning agent typically ranges from 0.01% to 10%, preferably from 0.1% to 8%, more preferably from 0.1% to 5%, more preferably from 0.2% to 3%. Examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions of the present invention preferably have a viscosity, as measured at 25°C, from 20 to 2,000,000 centistokes ("csk"), more preferably from 1,000 to 1,800,000 csk, even more preferably from 50,000 to 1,500,000 csk, more preferably from 100,000 to 1,500,000 csk.

The dispersed silicone conditioning agent particles typically have a volume average particle diameter ranging from 0.01µm to 50µm, as measured using the Horiba LA-910 Particle Size Analyzer. The Horiba LA-910 instrument uses the principles of low-angle Fraunhofer Diffraction and Light Scattering to measure the particle size and distribution in a dilute solution of particles. For small particle application to hair, the volume average particle diameters typically range from 0.01µm to 4µm, preferably from 0.01µm to 2µm, more preferably from 0.01µm to 0.5µm. For larger particle application to hair, the volume average particle diameters typically range from 4µm to 50µm, preferably from 6µm to 40µm, and more preferably from 10µm to 35µm.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

### a. Silicone oils

Silicone fluids include silicone oils, which are flowable silicone materials having a viscosity, as measured at 25°C, less than 1,000,000 csk, preferably from 5 csk to 1,000,000 csk, more preferably from 100 csk to 600,000 csk. Suitable silicone oils for use in the compositions of the present invention include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used.

Silicone oils include polyalkyl or polyaryl siloxanes which conform to the following Formula (III): 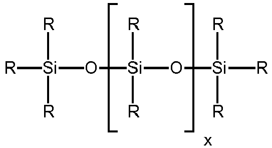 wherein R is aliphatic, preferably alkyl or alkenyl, or aryl, R can be substituted or unsubstituted, and x is an integer from 1 to 8,000. Suitable R groups for use in the compositions of the present invention include: alkoxy, aryloxy, alkaryl, arylalkyl, arylalkenyl, alkamino, and ether-substituted, hydroxyl-substituted, and halogen-substituted aliphatic and aryl groups. Suitable R groups also include cationic amines and quaternary ammonium groups.

Preferred alkyl and alkenyl substituents are C₁ to C₅ alkyls and alkenyls, more preferably from C₁ to C₄, more preferably from C₁ to C₂. The aliphatic portions of other alkyl-, alkenyl-, or alkynyl-containing groups (such as alkoxy, alkaryl, and alkamino) can be straight or branched chains, and are preferably from C₁ to C₅, more preferably from C₁ to C₄, even more preferably from C₁ to C₃, more preferably from C₁ to C₂. As discussed above, the R substituents can also contain amino functionalities (e.g. alkamino groups), which can be primary, secondary or tertiary amines or quaternary ammonium. These include mono-, di- and tri- alkylamino and alkoxyamino groups, wherein the aliphatic portion chain length is preferably as described herein.

### b. Amino and Cationic silicones

Cationic silicone fluids suitable for use in the compositions of the present invention include those which conform to the general formula (V):

(R₁)ₐG₃₋a-Si-(-OSiG₂)ₙ-(-OSiG_{b}(R₁)_{2-b)m}-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 0; b is 0 or 1, preferably 1; n is a number from 0 to 1,999, preferably from 49 to 499; m is an integer from 1 to 2,000, preferably from 1 to 10; the sum of n and m is a number from 1 to 2,000, preferably from 50 to 500; R₁ is a monovalent radical conforming to the general formula CqH_{2q}L, wherein q is an integer having a value from 2 to 8 and L is selected from the following groups:
- N(R₂)CH₂-CH₂-N(R₂)₂
- N(R₂)₂
- N(R₂)₃A⁻
- N(R₂)CH₂-CH₂-NR₂H₂A⁻
wherein R₂ is hydrogen, phenyl, benzyl, or a saturated hydrocarbon radical, preferably an alkyl radical from C₁ to C₂₀, and A⁻ is a halide ion.

An especially preferred cationic silicone corresponding to formula (V) is the polymer known as "trimethylsilylamodimethicone", which is shown below in formula (VI):

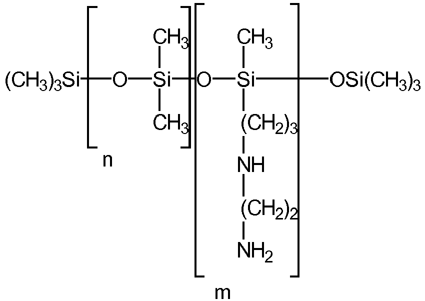

Other silicone cationic polymers which may be used in the compositions of the present invention are represented by the general formula (VII): 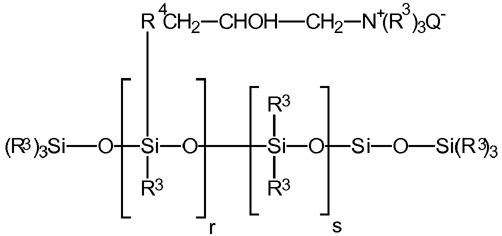 wherein R³ is a monovalent hydrocarbon radical from C₁ to C₁₈, preferably an alkyl or alkenyl radical, such as methyl; R₄ is a hydrocarbon radical, preferably a C₁ to C₁₈ alkylene radical or a C₁₀ to C₁₈ alkyleneoxy radical, more preferably a C₁ to C₈ alkyleneoxy radical; Q⁻ is a halide ion, preferably chloride; r is an average statistical value from 2 to 20, preferably from 2 to 8; s is an average statistical value from 20 to 200, preferably from 20 to 50. A preferred polymer of this class is known as UCARE SILICONE ALE 56™, available from Union Carbide.

### c. Silicone gums

Other silicone fluids suitable for use in the compositions of the present invention are the insoluble silicone gums. These gums are polyorganosiloxane materials having a viscosity, as measured at 25°C, of greater than or equal to 1,000,000 csk. Silicone gums are described in U.S. Pat. No. 4,152,416; Noll and Walter, Chemistry and Technology of Silicones, New York: Academic Press (1968); and in General Electric Silicone Rubber Product Data Sheets SE 30, SE 33, SE 54 and SE 76. Specific examples of silicone gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

### d. High refractive index silicones

Other non-volatile, insoluble silicone fluid conditioning agents that are suitable for use in the compositions of the present invention are those known as "high refractive index silicones," having a refractive index of at least 1.46, preferably at least 1.48, more preferably at least 1.52, more preferably at least 1.55. The refractive index of the polysiloxane fluid will generally be less than 1.70, typically less than 1.60. In this context, polysiloxane "fluid" includes oils as well as gums.

The high refractive index polysiloxane fluid includes those represented by general Formula (III) above, as well as cyclic polysiloxanes such as those represented by Formula (VIII) below: 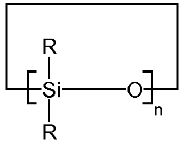 wherein R is as defined above, and n is a number from 3 to 7, preferably from 3 to 5.

The high refractive index polysiloxane fluids contain an amount of aryl-containing R substituents sufficient to increase the refractive index to the desired level, which is described herein. Additionally, R and n must be selected so that the material is non-volatile.

Aryl-containing substituents include those which contain alicyclic and heterocyclic five and six member aryl rings and those which contain fused five or six member rings. The aryl rings themselves can be substituted or unsubstituted.

Generally, the high refractive index polysiloxane fluids will have a degree of aryl-containing substituents of at least 15%, preferably at least 20%, more preferably at least 25%, even more preferably at least 35%, most preferably at least 50%. Typically, the degree of aryl substitution will be less than 90%, more generally less than 85%, preferably from 55% to 80%.

Preferred high refractive index polysiloxane fluids have a combination of phenyl or phenyl derivative substituents (more preferably phenyl), with alkyl substituents, preferably C₁-C₄ alkyl (more preferably methyl), hydroxy, or C₁-C₄ alkylamino (especially -R¹NHR²NH2 wherein each R¹ and R² independently is a C₁-C₃ alkyl, alkenyl, and/or alkoxy).

When high refractive index silicones are used in the compositions of the present invention, they are preferably used in solution with a spreading agent, such as a silicone resin or a surfactant, to reduce the surface tension by a sufficient amount to enhance spreading and thereby enhance the glossiness (subsequent to drying) of hair treated with the compositions.

Silicone fluids suitable for use in the compositions of the present invention are disclosed in U.S. Pat. No. 2,826,551, U.S. Pat. No. 3,964,500, U.S. Pat. No. 4,364,837, British Pat. No. 849,433, and *Silicon Compounds,* Petrarch Systems, Inc. (1984).

### e. Silicone resins

Silicone resins may be included in the silicone conditioning agent of the compositions of the present invention. These resins are highly cross-linked polymeric siloxane systems. The crosslinking is introduced through the incorporation of trifunctional and tetrafunctional silanes with monofunctional or difunctional, or both, silanes during manufacture of the silicone resin.

Silicone materials and silicone resins in particular, can conveniently be identified according to a shorthand nomenclature system known to those of ordinary skill in the art as "MDTQ" nomenclature. Under this system, the silicone is described according to presence of various siloxane monomer units which make up the silicone. Briefly, the symbol M denotes the monofunctional unit (CH₃)₃SiO_{0.5}; D denotes the difunctional unit (CH₃)₂SiO; T denotes the trifunctional unit (CH₃)SiO_{1.5}; and Q denotes the quadra- or tetra-functional unit SiO₂. Primes of the unit symbols (e.g. M', D', T', and Q') denote substituents other than methyl, and must be specifically defined for each occurrence.

Preferred silicone resins for use in the compositions of the present invention include MQ, MT, MTQ, MDT and MDTQ resins. Methyl is a preferred silicone substituent. Especially preferred silicone resins are MQ resins, wherein the M:Q ratio is from 0.5:1.0 to 1.5:1.0 and the average molecular weight of the silicone resin is from 1000 to 10,000.

The weight ratio of the non-volatile silicone fluid, having refractive index below 1.46, to the silicone resin component, when used, is preferably from 4:1 to 400:1, more preferably from 9:1 to 200:1, more preferably from 19:1 to 100:1, particularly when the silicone fluid component is a polydimethylsiloxane fluid or a mixture of polydimethylsiloxane fluid and polydimethylsiloxane gum as described herein. Insofar as the silicone resin forms a part of the same phase in the compositions hereof as the silicone fluid, i.e. the conditioning active, the sum of the fluid and resin should be included in determining the level of silicone conditioning agent in the composition.

### 2. Organic conditioning oils

The conditioning component of the compositions of the present invention may also comprise from 0.05% to 3%, preferably from 0.08% to 1.5%, more preferably from 0.1% to 1%, of at least one organic conditioning oil as the conditioning agent, either alone or in combination with other conditioning agents, such as the silicones (described herein).

### a. Hydrocarbon oils

Suitable organic conditioning oils for use as conditioning agents in the compositions of the present invention include hydrocarbon oils having at least 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from C₁₂ to C₁₉. Branched chain hydrocarbon oils, including hydrocarbon polymers, typically will contain more than 19 carbon atoms.

Specific examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation. The concentration of such hydrocarbon oils in the composition preferably range from 0.05% to 20%, more preferably from 0.08% to 1.5%, and even more preferably from 0.1% to 1%.

### b. Polyolefins

Organic conditioning oils for use in the compositions of the present invention can also include liquid polyolefins, more preferably liquid poly-α-olefins, more preferably hydrogenated liquid poly-α-olefins. Polyolefins for use herein are prepared by polymerization of C₄ to C₁₄ olefenic monomers, preferably from C₆ to C₁₂.

Examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents. Preferred hydrogenated α-olefin monomers include: 1-hexene to 1-hexadecenes, 1-octene to 1-tetradecene, and mixtures thereof.

### c. Fatty Esters

Other suitable organic conditioning oils for use as the conditioning agent in the compositions of the present invention include fatty esters having at least 10 carbon atoms. These fatty esters include esters with hydrocarbyl chains derived from fatty acids or alcohols (e.g. monoesters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.).

Specific examples of preferred fatty esters include: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the compositions of the present invention are monocarboxylic acid esters of the general formula R'COOR, wherein R' and R are alkyl or alkenyl radicals, and the sum of carbon atoms in R' and R is at least 10, preferably at least 22.

Still other fatty esters suitable for use in the compositions of the present invention are di- and tri-alkyl and alkenyl esters of carboxylic acids, such as esters of C₄ to C₈ dicarboxylic acids (e.g. C₁ to C₂₂ esters, preferably C₁ to C₆, of succinic acid, glutaric acid, and adipic acid). Specific examples of di- and tri- alkyl and alkenyl esters of carboxylic acids include isocetyl stearyol stearate, diisopropyl adipate, and tristearyl citrate.

Other fatty esters suitable for use in the compositions of the present invention are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Still other fatty esters suitable for use in the compositions of the present invention are glycerides, including mono-, di-, and tri-glycerides, preferably di- and tri-glycerides, more preferably triglycerides. For use in the compositions described herein, the glycerides are preferably the mono-, di-, and tri-esters of glycerol and long chain carboxylic acids, such as C₁₀ to C₂₂ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include triolein and tristearin glyceryl dilaurate.

Other fatty esters suitable for use in the compositions of the present invention are water insoluble synthetic fatty esters. Some preferred synthetic esters conform to the general Formula (IX): 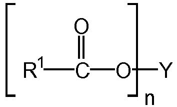 wherein R¹ is a C₇ to C₉ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from 2 to 20 carbon atoms, preferably from 3 to 14 carbon atoms. Other preferred synthetic esters conform to the general Formula (X): 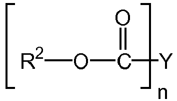 wherein R² is a C₈ to C₁₀ alkyl, alkenyl, hydroxyalkyl or hydroxyalkenyl group; preferably a saturated alkyl group, more preferably a saturated, linear, alkyl group; n and Y are as defined above in Formula (X).

Specific examples of suitable synthetic fatty esters for use in the compositions of the present invention include: P-43 (C₈-C₁₀ triester of trimethylolpropane), MCP-684 (tetraester of 3,3 diethanol-1,5 pentadiol), MCP 121 (C₈-C₁₀ diester of adipic acid), all of which are available from Mobil Chemical Company.

### 3. Other conditioning agents

Also suitable for use in the compositions herein are the conditioning agents described by the Procter & Gamble Company in U.S. Pat. Nos. 5,674,478, and 5,750,122. Also suitable for use herein are those conditioning agents described in U.S. Pat. Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

### 4. Additional Components

The compositions of the present invention may further include a variety of additional useful components. Preferred additional components include those discussed below:

### 1. Other Anti-Microbial Actives

The compositions of the present invention may further include one or more anti-fungal or anti-microbial actives in addition to the metal pyrithione salt actives. Suitable anti-microbial actives include coal tar, sulfur, whitfield's ointment, castellani's paint, aluminum chloride, gentian violet, octopirox (piroctone olamine), ciclopirox olamine, undecylenic acid and it's metal salts, potassium permanganate, selenium sulfide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Preferred anti-microbials include itraconazole, ketoconazole, selenium sulphide and coal tar.

### a. Azoles

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from 0.01% to 5%, preferably from 0.1% to 3%, and more preferably from 0.3% to 2%, by weight of the composition. Especially preferred herein is ketoconazole.

### b. Selenium Sulfide

Selenium sulfide is a particulate anti-dandruff agent suitable for use in the anti-microbial compositions of the present invention, effective concentrations of which range from 0.1% to 4%, by weight of the composition, preferably from 0.3% to 2.5%, more preferably from 0.5% to 1.5%. Selenium sulfide is generally regarded as a compound having one mole of selenium and two moles of sulfur, although it may also be a cyclic structure that conforms to the general formula SeₓS_{y}, wherein x + y = 8. Average particle diameters for the selenium sulfide are typically less than 15µm, as measured by forward laser light scattering device (e.g. Malvern 3600 instrument), preferably less than 10 µm. Selenium sulfide compounds are described, for example, in U.S. Pat. No. 2,694,668; U.S. Pat. No. 3,152,046; U.S. Pat. No. 4,089,945; and U.S. Pat. No. 4,885,107.

### c. Sulfur

Sulfur may also be used as a particulate anti-microbial/anti-dandruff agent in the anti- microbial compositions of the present invention. Effective concentrations of the particulate sulfur are typically from 1% to 4%, by weight of the composition, preferably from 2% to 4%.

### d. Keratolytic Agents

The present invention may further comprise one or more keratolytic agents such as Salicylic Acid.

Additional anti-microbial actives of the present invention may include extracts of melaleuca (tea tree) and charcoal. The present invention may also comprise combinations of anti- microbial actives. Such combinations may include octopirox and zinc pyrithione combinations, pine tar and sulfur combinations, salicylic acid and zinc pyrithione combinations, octopirox and climbasole combinations, and salicylic acid and octopirox combinations, and mixtures thereof.

### 2. Hair loss prevention and Hair Growth Agents

The present invention may further comprise materials useful for hair loss prevention and hair growth stimulants or agents. Examples of such agents are Anti-Androgens such as Propecia, Dutasteride, RU5884; Anti-Inflammatories such as Glucocortisoids, Macrolides, Macrolides; Anti-Microbials such as Zinc pyrithione, Ketoconazole, Acne Treatments; Immunosuppressives such as FK-506, Cyclosporin; Vasodilators such as minoxidil, Aminexil® and combinations thereof.

### 3. Sensates

The present invention may further comprise topical sensate materials such as terpenes, vanilloids, alkyl amides, natural extracts and combinations thereof. Terpenes can include menthol and derivatives such as menthyl lactate, ethyl menthane carboxamide, and menthoyxypropanediol. Other terpenes can include camphor, eucalyptol, carvone, thymol and combinations thereof. Vanilloids can include capsaicin, zingerone, eugenol, and vanillyl butyl ether. Alkyl amides can include spilanthol, hydroxy alpha-sanschool, pellitorine and combinations thereof. Natural extracts can include peppermint oil, eucalyptol, rosemary oil, ginger oil, clove oil, capsicum, jambu extract, cinnamon oil, laricyl and combinations thereof. Additional topical sensate materials can include methyl salicylate, anethole, benzocaine, lidocane, phenol, benzyl nicotinate, nicotinic acid, cinnamic aldehyde, cinnamyl alcohol, piperine, and combinations thereof.

### 4. Humectant

The compositions of the present invention may contain a humectant. The humectants herein are selected from the group consisting of polyhydric alcohols, water soluble alkoxylated nonionic polymers, and mixtures thereof. The humectants, when used herein, are preferably used at levels of from 0.1% to 20%, more preferably from 0.5% to 5%.

Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosine phosphate, sodium lactate, pyrrolidone carbonate, glucosamine, cyclodextrin, and mixtures thereof.

Water soluble alkoxylated nonionic polymers useful herein include polyethylene glycols and polypropylene glycols having a molecular weight of up to 1000 such as those with CTFA names PEG-200, PEG-400, PEG-600, PEG-1000, and mixtures thereof.

### 5. Suspending Agent

The compositions of the present invention may further comprise a suspending agent at concentrations effective for suspending water-insoluble material in dispersed form in the compositions or for modifying the viscosity of the composition. Such concentrations range from 0.1% to 10%, preferably from 0.3% to 5.0%.

Suspending agents useful herein include anionic polymers and nonionic polymers. Useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Commercially available viscosity modifiers highly useful herein include Carbomers with tradenames Carbopol 934, Carbopol 940, Carbopol 950, Carbopol 980, and Carbopol 981, all available from B. F. Goodrich Company, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxyethyl cellulose with tradename NATROSOL, hydroxypropyl cellulose with tradename KLUCEL, cetyl hydroxyethyl cellulose with tradename POLYSURF 67, all supplied by Hercules, ethylene oxide and/or propylene oxide based polymers with tradenames CARBOWAX PEGs, POLYOX WASRs, and UCON FLUIDS, all supplied by Amerchol.

Other optional suspending agents include crystalline suspending agents which can be categorized as acyl derivatives, long chain amine oxides, and mixtures thereof. These suspending agents are described in U.S. Pat. No. 4,741,855. These preferred suspending agents include ethylene glycol esters of fatty acids preferably having from 16 to 22 carbon atoms. More preferred are the ethylene glycol stearates, both mono and distearate, but particularly the distearate containing less than 7% of the mono stearate. Other suitable suspending agents include alkanol amides of fatty acids, preferably having from 16 to 22 carbon atoms, more preferably 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin) a commercial example of which is Thixin R available from Rheox, Inc. Long chain acyl derivatives, ethylene glycol esters of long chain carboxylic acids, long chain amine oxides, and alkanol amides of long chain carboxylic acids in addition to the preferred materials listed above may be used as suspending agents.

Other long chain acyl derivatives suitable for use as suspending agents include N,N-dihydrocarbyl amido benzoic acid and soluble salts thereof (e.g., Na, K), particularly N,N-di(hydrogenated) C.sub.16, C.sub.18 and tallow amido benzoic acid species of this family, which are commercially available from Stepan Company (Northfield, Ill., USA).

Examples of suitable long chain amine oxides for use as suspending agents include alkyl dimethyl amine oxides, e.g., stearyl dimethyl amine oxide.
Other suitable suspending agents include primary amines having a fatty alkyl moiety having at least 16 carbon atoms, examples of which include palmitamine or stearamine, and secondary amines having two fatty alkyl moieties each having at least 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

### 6. Other Optional Components

The compositions of the present invention may contain also vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names.

The compositions of the present invention may also contain antimicrobial agents which are useful as cosmetic biocides and antidandruff agents including: water soluble components such as piroctone olamine, water insoluble components such as 3,4,4'- trichlorocarbanilide (triclocarban), triclosan and zinc pyrithione.

The compositions of the present invention may also contain chelating agents.

### H. pH

Preferably, the pH of the compositions of the present invention range from 2 to 10, preferably from 3 to 9.5, more preferably from 4 to 9.

### I. Augmentation of ZPT Activity

Zinc pyrithione (ZPT) is an effective antifungal material. Antifungal activity is routinely assessed by quantifying the minimum inhibitory concentration (MIC) against a specific organism under specific conditions.¹ The lower the MIC, the more potent the activity against that organism. In some cases, materials can augment the activity of antifungal actives. As used herein, the term augmentation is intended to mean enhancement of the antifungal activity of a ZPT-containing composition by the augmenting material to an extent sufficient to permit the use of a lower amount of ZPT without reducing the antifungal inhibition provided by the composition, as compared to the antifungal inhibition that is provided by an otherwise-identical ZPT-containing composition that is free of the augmenting material. Augmenting agents are generally specific to the material being augmented, forming a unique combination and often because there is a specific chemical or biological mechanism required to achieve the augmentation effect.

An "augmentation factor" is defined, which is the ratio of the MIC with and without an augmenting agent (at a specified concentration): $AugmentationFactor = \frac{MIC of ZPT Alone}{MIC of ZPT with 5 ppm augmenting agent}$

An effective augmenting agent would have an augmentation factor greater than 1, preferably greater than 1.3 and even more preferably greater than 1.5.

Examples of some evaluations include the following:

| | MIC of ZPT alone | MIC of ZPT with 5ppm augmenting material | Augmentation Factor | Augmentation Benefit |
|---|---|---|---|---|
| Zinc Stearate | 8.0 | 8.0 | 1.0 | No |
| Zinc Sulfide | 8.0 | 8.0 | 1.0 | No |
| Basic zinc carbonate | 8.0 | 2.0 | 4.0 | Yes |

¹ The Minimum Inhibitory Concentration is indicative of anti-fungal efficacy. Generally, the lower the value of the composition, the better its anti-fungal efficacy, due to increased inherent ability of the anti-dandruff agent to inhibit the growth of microorganisms.

### MIC measurement method

*Malassezia furfur* was grown in a flask containing mDixon medium (see E. Gueho, et al. Antoinie Leeuwenhoek (1996), no. 69, 337-55). Dilutions of solubilized anti-microbial active were then added to test tubes containing molten mDixon agar. *M. furfur* inoculum was added to each tube of molten agar, the tube vortexed, and the contents poured into separate sterile petri dishes. After the plates are incubated, they were observed for visible *M. furfur* growth. The lowest tested dilution of anti-microbial active that yields no growth is defined as the Minimal Inhibitory Concentration (MIC).

### Equipment/Reagents

| | |
|---|---|
| Microbe | *Malassezia furfur* (ATCC 14521) |
| Erlenmeyer flask | 250ml |
| Agar medium | 9.5ml mDixon agar per concentration per active tested |
| Solvent | water, dimethyl sulfonyl oxide ("DMSO") |
| | Zinc pyridinethione ZPT having an average particle size of 2.5µm. |
| Test tubes | 2 tubes per anti-microbial active per concentration per active tested, sterilized, size = 18mm x 150mm |
| Petri dishes | 2 dishes per anti-microbial active per concentration per active tested, sterilized, size = 15mm x 100mm |

### Experimental procedure

1) *Malassezia furfur* was grown in a 250 ml Erlenmeyer flask containing 100 ml "mDIXON" medium at 320 rpm and 30°C until turbid.
2) Selected dilutions were prepared using an appropriate dilution series, of the anti-microbial active or combination in solvent, which allowed the sample active to be solubilized prior to addition to the final test agar. For each concentration of the ZPT samples, the solvent was "DMSO"; for other samples, the solvent was water or "DMSO" or other suitable solvent.
3) 0.25 ml dilutions of anti-microbial active were added to test tubes containing 9.5 ml molten "mDIXON" agar (held at 45°C in a water bath).
4) 0.25 *M. furfur* inoculum (adjusted to 5 x 10⁵ cfu/ml by direct count) was added to each test tube of molten agar.
5) Each tube was vortexed, and the contents poured into separate petri dishes.
6) After the agar solidified, the plates inverted and incubated at 30° for 5 days.
7) The plates were then observed for visible *M. furfur* growth.

### J. Methods of Use

The compositions of the present invention may be used in direct application to the skin or in a conventional manner for cleansing skin and hair and controlling microbial infection (including fungal, viral, or bacterial infections) on the skin or scalp. The compositions herein are useful for cleansing the hair and scalp, and other areas of the body such as underarm, feet, and groin areas and for any other area of skin in need of treatment. The present invention may be used for treating or cleansing of the skin or hair of animals as well. An effective amount of the composition, typically from 1g to 50g, preferably from 1g to 20g of the composition, for cleansing hair, skin or other area of the body, is topically applied to the hair, skin or other area that has preferably been wetted, generally with water, and then rinsed off. Application to the hair typically includes working the shampoo composition through the hair.

A preferred method for providing anti-microbial (especially anti-dandruff) efficacy with a shampoo embodiment comprises the steps of: (a) wetting the hair with water, (b) applying an effective amount of the anti-microbial shampoo composition to the hair, and (c) rinsing the anti-microbial shampoo composition from the hair using water. These steps may be repeated as many times as desired to achieve the cleansing, conditioning, and anti-microbial/anti-dandruff benefits sought.

It is also contemplated that when the anti-microbial active employed is zinc pyrithione, and/or if other optional hair growth regulating agents are employed, the anti-microbial compositions of the present invention, may, provide for the regulation of growth of the hair. The method of regularly using such shampoo compositions comprises repeating steps a, b, and c (above).

A further embodiment of the present invention comprises a method comprising the steps of (a) wetting the hair with water, (b) applying an effective amount of a shampoo composition comprising a zinc-containing layered material, (c) rinsing the shampoo compositions from the hair using water; (d) applying an effective amount of a conditioner composition comprising a zinc-containing layered material according to the present invention; (e) rinsing the conditioner composition from the hair using water. A preferred embodiment of the above mentioned method includes a shampoo composition comprising zinc pyrithione and a conditioner composition comprising basic zinc carbonate.

A further embodiment of the present invention comprises a method of treating athlete's foot comprising the use of the composition according to the present invention, a method of treating microbial infections comprising the use of composition as described herein, method of improving the appearance of a scalp comprising the use of the composition according present invention, a method of treating fungal infections comprising the use of the composition according to the present invention, a method of treating dandruff comprising the use of the composition of the present invention, a method of treating diaper dermatitis and candidiasis comprising the use of the compositions of the present invention as described herein, a method of treating tinea capitis comprising the use of the composition according to the present invention, a method of treating yeast infections comprising the use of the composition according to the present invention, a method of treating onychomycosis comprising the use of the composition according to the present invention.

### K. Examples

The following examples further describe and demonstrate the preferred embodiments within the scope of the present invention..

The composition of the invention can be made by mixing one or more selected metal ion sources and one or more metal salts of pyrithione in an appropriate media or carrier, or by adding the individual components separately to the skin or hair cleansing compositions. Useful carriers are discussed more fully above.

### 1. Topical Compositions

All exemplified compositions can be prepared by conventional formulation and mixing techniques. Component amounts are listed as weight percents and exclude minor materials such as diluents, filler, and so forth. The listed formulations, therefore, comprise the listed components and any minor materials associated with such components. As used herein, "minors" refers to those optional components such as preservatives, viscosity modifiers, pH modifiers, fragrances, foam boosters, and the like. As is apparent to one of ordinary skill in the art, the selection of these minors will vary depending on the physical and chemical characteristics of the particular ingredients selected to make the present invention as described herein. Other modifications can be undertaken by the skilled artisan without departing from the spirit and scope of this invention. These exemplified embodiments of the anti-microbial shampoo, anti-microbial conditioner, anti-microbial leave-on tonic, and anti-microbial foot powder compositions of the present invention provide excellent anti-microbial efficacy.

### L. Methods of Manufacture For Shampoo Compositions

The compositions of the present invention may be prepared by any known or otherwise effective technique, suitable for providing an anti-microbial composition provided that the resulting composition provides the excellent anti-microbial benefits described herein. Methods for preparing the anti-dandruff and conditioning shampoo embodiments of the present invention include conventional formulation and mixing techniques. A method such as that described in U.S. Pat. No. 5,837,661, could be employed, wherein the anti-microbial agent of the present invention would typically be added in the same step as the silicone premix is added in the U.S. Pat. No. 5,837,661 description.

### Antimicrobial Shampoo - Examples 4, 6, 15, 16, 17, 19, 20, 22, 26, 28, 31, 32, 33, 34, 37 and 39

A suitable method for preparing the anti-microbial shampoo compositions described in Examples 4, 6, 15, 16, 17, 19, 20, 22, 26, 28, 31, 32, 33, 34, 37 and 39 (below) follows:
About one-third to all of the sodium laureth sulfate (added as 29wt% solution) and acid was added to a jacketed mix tank and heated to 60°C to 80°C with slow agitation to form a surfactant solution. The pH of this solution was 3 to 7. Sodium benzoate, Cocoamide MEA and fatty alcohols, (where applicable), was added to the tank and allowed to disperse. Ethylene glycol distearate ("EGDS") was added to the mixing vessel and allowed to melt (where applicable). After the EGDS was melted and dispersed, Kathon CG was added to the surfactant solution. The resulting mixture was cooled to 25°C to 40°C and collected in a finishing tank. As a result of this cooling step, the EGDS crystallized to form a crystalline network in the product (where applicable). The remainder of the sodium laureth sulfate and other components, including the silicone and anti-microbial agent(s), were added to the finishing tank with agitation to ensure a homogeneous mixture. Polymers (cationic or nonionic) were dispersed in water or oils as an 0.1% to 10% dispersion and/or solution and could be added to the main mix, final mix, or both. Basic Zinc Carbonate or other zinc-containing layered material could be added to a premix of surfactants or water with or without the aid of a dispersing agent via conventional powder incorporation and mixing techniques into the final mix. Once all components had been added, additional viscosity modifiers, such as sodium chloride and/or sodium xylenesulfonate could be added, as needed, to adjust product viscosity to the extent desired. Product pH was adjusted, using an acid such as hydrochloric acid, to an acceptable value.

### Antimicrobial Shampoo - Proposed Examples 1-3, 5, 7-14, 18, 21, 23-25, 27, 29-30, 35-36, and 38

A suitable method for preparing the anti-microbial shampoo compositions described in Proposed Examples 1-3, 5, 7-14, 18, 21, 23-25, 27, 29-30, 35-36, and 38
(below) follows:
About one-third to all of the sodium laureth sulfate (added as 29wt% solution) and acid are added to a jacketed mix tank and heated to 60°C to 80°C with slow agitation to form a surfactant solution. The pH of this solution is 3 to 7. Sodium benzoate, Cocoamide MEA and fatty alcohols, (where applicable), are added to the tank and allowed to disperse. Ethylene glycol distearate ("EGDS") is added to the mixing vessel and allowed to melt (where applicable). After the EGDS is melted and dispersed, Kathon CG is added to the surfactant solution. The resulting mixture is cooled to 25°C to 40°C and collected in a finishing tank. As a result of this cooling step, the EGDS crystallizes to form a crystalline network in the product (where applicable). The remainder of the sodium laureth sulfate and other components, including the silicone and anti-microbial agent(s), are added to the finishing tank with agitation to ensure a homogeneous mixture. Polymers (cationic or nonionic) are dispersed in water or oils as an 0.1% to 10% dispersion and/or solution and can be added to the main mix, final mix, or both. Basic Zinc Carbonate or other zinc-containing layered material can be added to a premix of surfactants or water with or without the aid of a dispersing agent via conventional powder incorporation and mixing techniques into the final mix. Once all components have been added, additional viscosity modifiers, such as sodium chloride and/or sodium xylenesulfonate may be added, as needed, to adjust product viscosity to the extent desired. Product pH can be adjusted, using an acid such as hydrochloric acid, to an acceptable value.

### Shampoo Compositions - Examples 4, 6, 15, 16, 17, 19, 20, 22, 26, 28, 31, 32, 33, 34, 37 and 39 And Proposed Examples 1-3, 5, 7-14, 18, 21, 23-25, 27, 29-30, 35-36, and 38

| **Components** | **Example 1** | **Example 2** | **Example 3** | **Example 4** | **Example 5** | **Example 6** | **Example 7** | **Example 8** | **Example 9** |
|---|---|---|---|---|---|---|---|---|---|
| Sodium Laureth Sulfate | 10.00 | 10.00 | 10.00 | 10.00 | | 10.00 | 10.00 | 10.00 | 10.00 |
| Sodium Lauryl Sulfate | 6.00 | 6.00 | 6.00 | 6.00 | 2.00 | 6.00 | 6.00 | 6.00 | 6.00 |
| Decyl Glucoside | | | | | 10.00 | | | | |
| EGDS | 1.50 | 1.50 | 1.50 | 1.50 | 1.50 | 1.50 | 1.50 | 1.50 | 1.50 |
| CMEA | 0.800 | 0.800 | 0.800 | 0.800 | 0.800 | 0.800 | 0.800 | 0.800 | 0.800 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.500 | 0.500 | 0.500 | 0.500 | 0.500 | 0.500 | 0.500 | 0.500 |
| Dimethicone (2) | 0.85 | 0.85 | 0.85 | 0.85 | 0.85 | 0.85 | 0.85 | 0.85 | 0.85 |
| ZPT (3) | 1.00 | 1.00 | 1.00 | 1.00 | 1.00 | 1.00 | 1.00 | 1.50 | 1.75 |
| Basic Zinc Carbonate (4) | | | | | 1.61 | 1.61 | 3.22 | 1.61 | 1.61 |
| Zinc Hydroxy Sulfate (5) | 2.00 | | | | | | | | |
| Zinc Hydroxy Nitrate (5) | | 1.88 | | | | | | | |
| Zinc Hydroxy Chloride (5) | | | 1.63 | | | | | | |
| Zinc Hydroxy Lauryl Sulfate (5) | | | | 2.40 | | | | | |
| Hydrochloric Acid (6) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| **(1)** Guar having a molecular weight of 400,000, and having a charge density of 0.84 meq/g, available from Aqualon. **(2)** Viscasil 330M available from General Electric Silicones **(3)** ZPT having an average particle size of 2.5µm, available under the OMADINE trademark from Arch Chemicals, Inc. **(4)** Basic Zinc Carbonate Available from Bruggemann Chemical **(5)** Materials made by reported methods in Lagaly, G.; et al. Inorg. Chem. 1993, 32, 1209-1215 & Morioka, H.; et al. Inorg. Chem. 1999, 38, 4211-4216 **(6)** 6N HCl, available from J.T. Baker, adjustable to achieve target pH | | | | | | | | | |

| **Components** | **Example 10** | **Example 11** | **Example 12** | **Example 13** | **Example 14** | **Example 15** | **Example 16** | **Example 17** | **Example 18** |
|---|---|---|---|---|---|---|---|---|---|
| Sodium Laureth Sulfate | 10.00 | 10.00 | 10.00 | 10.00 | 10.00 | 10.00 | 10.00 | 10.00 | 10.00 |
| Sodium Lauryl Sulfate | 6.00 | 6.00 | 6.00 | 6.00 | 6.00 | 6.00 | 6.00 | 6.00 | 6.00 |
| Cocamidopropyl Betaine | | | | | | | | | |
| EGDS | 1.50 | 1.50 | 1.50 | 1.50 | 1.50 | 1.50 | 1.50 | 1.50 | 1.50 |
| CMEA | 0.800 | 0.800 | 0.800 | 0.800 | 0.800 | 0.800 | 0.800 | 0.800 | 0.800 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.500 | 0.500 | 0.500 | 0.500 | 0.500 | 0.500 | 0.500 | 0.500 |
| Dimethicone (2) | 0.85 | 0.85 | 0.85 | 0.85 | 0.85 | 1.00 | 1.35 | 1.60 | |
| Dimethicone (3) | | | | | | | | | 1.00 |
| ZPT (4) | 2.00 | 0.50 | 2.00 | 2.00 | 2.00 | 1.00 | 1.00 | 1.00 | 1.00 |
| Basic Zinc Carbonate (5) | 3.22 | 1.61 | 1.61 | 0.40 | 0.80 | 1.61 | 1.61 | 1.61 | 1.61 |
| Hydrochloric Acid (6) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.300 | 0.750 | 0.750 | 0.750 | 0.750 | 0.750 | 0.750 | 1.00 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| **(1)** Guar having a molecular weight of 400,000, and having a charge density of 0.84 meq/g, available from Aqualon. **(2)** Viscasil 330M available from General Electric Silicones **(3)** 1664 Emulsion available from Dow Corning **(4)** ZPT having an average particle size of 2.5µm, available under the OMADINE trademark from Arch Chemicals, Inc. **(5)** Basic Zinc Carbonate Available from Bruggemann Chemical **(6)** 6N HCl, available from J.T. Baker, adjustable to achieve target pH | | | | | | | | | |

| **Components** | **Example 19** | **Example 20** | **Example 21** | **Example 22** | **Example 23** | **Example 24** | **Example 25** | **Example 26** | **Example 27** |
|---|---|---|---|---|---|---|---|---|---|
| Sodium Laureth Sulfate | 10.00 | 10.00 | 10.00 | 10.00 | 10.00 | 10.00 | 10.00 | 10.00 | 10.00 |
| Sodium Lauryl Sulfate | 6.00 | 6.00 | 6.00 | 6.00 | 6.00 | 6.00 | 6.00 | 6.00 | 6.00 |
| EGDS | 1.50 | 1.50 | 1.50 | 1.50 | 1.50 | 1.50 | 1.50 | 1.50 | 1.50 |
| CMEA | 1.600 | 1.600 | 0.800 | 0.800 | 1.600 | 0.800 | 0.800 | 0.800 | 0.800 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.400 | | | | 0.500 | 0.500 | 0.500 | 0.500 |
| Guar Hydroxy Propyl Trimonium Chloride (2) | | | | 0.500 | | | | | |
| Guar Hydroxy Propyl Trimonium Chloride (3) | | | 0.500 | | 0.500 | | | | |
| PEG-7M (4) | | | | | | 0.200 | | | 0.100 |
| PEG-14M (5) | | | | | | | 0.200 | | |
| PEG-45M (6) | | | | | | | | 0.200 | |
| Dimethicone (7) | 0.85 | 0.85 | 0.85 | 0.85 | 0.85 | 0.85 | 0.85 | 0.85 | 0.85 |
| ZPT (8) | 1.00 | 1.00 | 1.00 | 1.00 | 1.00 | 1.00 | 1.00 | 1.00 | 1.00 |
| Basic Zinc Carbonate (9) | 1.61 | 1.61 | 1.61 | 1.61 | 1.61 | 1.61 | 1.61 | 1.61 | 1.61 |
| Hydrochloric Acid (10) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| (1) Guar having a molecular weight of 400,000, and having a charge density of 0.84 meq/g, available from Aqualon. (2) Guar having a molecular weight of 600,000, and having a charge density of 2.0 meq/g, available from Aqualon (3) Jaguar C-17, available from Rhodia (4) Polyox WSR N-750, available from Amerchol (5) Polyox WSR N-3000, available from Amerchol **(6)** Polyox WSR N-60K, available from Amerchol **(7)** Viscasil 330M available from General Electric Silicones **(8)** ZPT having an average particle size of 2.5µm, available under the OMADINE trademark from Arch Chemicals, Inc. **(9)** Basic Zinc Carbonate Available from Bruggemann Chemical **(10)** 6N HCl, available from J.T. Baker, adjustable to achieve target pH | | | | | | | | | |

| Components | **Example 28** | **Example 29** | **Example 30** | **Example 31** | **Example 32** | **Example 33** | **Example 34** | **Example 35** | **Example 36** |
|---|---|---|---|---|---|---|---|---|---|
| Sodium Laureth Sulfate | 10.00 | 12.50 | 14.00 | 10.00 | 10.00 | 10.00 | 10.00 | 10.00 | 10.00 |
| Sodium Lauryl Sulfate | 6.00 | 1.50 | 6.00 | 6.00 | 6.00 | 6.00 | 6.00 | 6.00 | 6.00 |
| Cocamidopropyl Betaine | 2.00 | 2.70 | | | | | | | |
| EGDS | 1.50 | 1.50 | 1.50 | 1.50 | 1.50 | 1.50 | 1.50 | 1.50 | 1.50 |
| CMEA | 0.800 | 0.800 | 0.800 | 1.600 | 1.600 | 1.600 | 1.600 | 0.800 | 0.800 |
| Cetyl Alcohol | 0.600 | 0.600 | | 0.600 | 0.600 | 0.600 | 0.600 | 0.600 | 0.600 |
| Guar Hydroxy Propyl Trimonium Chloride (1) | 0.500 | 0.500 | 0.500 | 0.500 | | | | | |
| Polyquaterium-10 (2) | | | | | | | | 0.500 | 0.500 |
| Polyquaterium-10 (3) | | | | | 0.500 | 0.500 | 0.400 | | |
| PEG-7M (4) | | | | 0.200 | | | | | 0.100 |
| Dimethicone (5) | 0.85 | 0.85 | 0.85 | 0.85 | 1.40 | 0.85 | 1.40 | 1.40 | 1.40 |
| ZPT (6) | 1.00 | 1.00 | 1.00 | 1.00 | 1.00 | 1.00 | 1.00 | 1.00 | 1.00 |
| Basic Zinc Carbonate (7) | 1.61 | 1.61 | 1.61 | 1.61 | 1.61 | 1.61 | 1.61 | 1.61 | 1.61 |
| Hydrochloric Acid (8) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| **(1)** Guar having a molecular weight of 400,000, and having a charge density of 0.84 meq/g, available from Aqualon. **(2)** UCARE Polymer JR 30M, available from Amerchol **(3)** UCARE Polymer LR 400, available from Amerchol **(4)** POLYOX WSR N-750, available from Amerchol **(5)** Viscasil 330M available from General Electric Silicones **(6)** ZPT having an average particle size of 2.5µm, available under the OMADINE trademark from Arch Chemicals, Inc. **(7)** Basic Zinc Carbonate Available from Bruggemann Chemical **(8)** 6N HCl, available from J.T. Baker, adjustable to achieve target pH | | | | | | | | | |

| Components | **Example 37** | **Example 38** | **Example 39** |
|---|---|---|---|
| Sodium Laureth Sulfate | 10.00 | 10.00 | 10.00 |
| Sodium Lauryl Sulfate | 6.00 | 6.00 | 6.00 |
| EGDS | 1.50 | 1.50 | 1.50 |
| CMEA | 1.600 | 1.600 | 1.600 |
| Cetyl Alcohol | 0.600 | 0.600 | 0.600 |
| Guar Hydroxy Propyl Trimonium Chloride (1) | | | 0.400 |
| Polyquaterium-10 (2) | 0.500 | 0.250 | 0.100 |
| PEG-7M (3) | 0.100 | | 0.100 |
| Dimethicone (4) | 0.85 | 0.85 | 0.85 |
| ZPT (5) | 1.00 | 1.00 | 1.00 |
| Basic Zinc Carbonate (6) | 1.61 | 1.61 | 1.61 |
| Hydrochloric Acid (7) | 0.42 | 0.42 | 0.42 |
| Magnesium Sulfate | 0.28 | 0.28 | 0.28 |
| Sodium Chloride | 0.800 | 0.800 | 0.800 |
| Sodium Xylenesulfonate | | | |
| Perfume | 0.750 | 0.750 | 0.750 |
| Sodium Benzoate | 0.250 | 0.250 | 0.250 |
| Kathon | 0.0008 | 0.0008 | 0.0008 |
| Benzyl Alcohol | 0.0225 | 0.0225 | 0.0225 |
| Water | Q.S. | Q.S. | Q.S. |

| | | | |
|---|---|---|---|
| **(1)** Guar having a molecular weight of 400,000, and having a charge density of 0.84 meq/g, available from Aqualon. **(2)** UCARE Polymer LR 400, available from Amerchol **(3)** POLYOX WSR N-750, available from Amerchol **(4)** Viscasil 330M available from General Electric Silicones **(5)** ZPT having an average particle size of 2.5 µm, available under the OMADINE trademark from Arch Chemicals, Inc. **(6)** Basic Zinc Carbonate Available from Bruggemann Chemical **(7)** 6N HCl, available from J.T. Baker, adjustable to achieve target pH | | | |

### Cleansing Compositions - Proposed Examples 40-44

A suitable method for preparing the anti-microbial cleansing compositions described in Proposed Examples 40-44 (below) follows:
Components 1-3, 7, and 8 are mixed with heating to 190F. Components 4, 10, 13 and 15 are mixed at room temperature in a separate pot. After the first mixture has reached 190F, it is added to the second mixture. After this mixture has cooled below 140 F, components 11 (& 5) is added. In a separate vessel at 160 F, the petrolatum and Basic Zinc Carbonate are mixed. When the aqueous phase has cooled below 110 F, the petrolatum/Basic Zinc Carbonate blend is added and agitated until smooth. Basic Zinc Carbonate can also be added to a premix of surfactants or water with or without the aid of a dispersing agent via conventional powder incorporation and mixing techniques into the cooled mixture. Finally the perfume is added.

| Components | **Example 40** | **Example 41** | **Example 42** | **Example 43** | **Example 44** |
|---|---|---|---|---|---|
| 1.Sodium Lauryl Sulfate | 4.000 | 4.000 | 4.000 | 4.000 | 4.000 |
| 2. Sodium Laureth Sulfate | 3.000 | 3.000 | 3.000 | 3.000 | 3.000 |
| 3. Sodium Laruroamphoacetate | 4.000 | 4.000 | 4.000 | 4.000 | 4.000 |
| 4. Sodium Lauroyl Sarcosinate | 2.000 | 2.000 | 2.000 | 2.000 | 2.000 |
| 5.Zinc Pyrithione (1) | 1.000 | 1.000 | 1.000 | 2.000 | 2.000 |
| 6 Basic Zinc Carbonate (2) | 1.610 | | | | |
| Zinc Hydroxy Sulfate (3) | | 2.000 | | | |
| Zinc Hydroxy Nitrate (3) | | | 1.880 | | |
| Zinc Hydroxy Chloride (3) | | | | 1.630 | |
| Zinc Hydroxy Lauryl Sulfate (3) | | | | | 2.400 |
| 7. Lauric Acid | 1.000 | 1.000 | 1.000 | 1.000 | 1.000 |
| 8. Trihydroxystearin | 0.650 | 0.650 | 0.650 | 0.650 | 0.650 |
| 9. Citric Acid | 0.750 | 0.750 | 0.750 | 0.750 | 0.750 |
| 10.Sodium Benzoate | 0.250 | 0.250 | 0.250 | 0.250 | 0.250 |
| 11. Glydant | 0.120 | 0.120 | 0.120 | 0.120 | 0.120 |
| 12. Perfume | 0.750 | 0.750 | 0.750 | 0.750 | 0.750 |
| 13. Polyquaterium-10 (4) | 0.750 | 0.750 | 0.750 | 0.750 | 0.750 |
| 14. Petrolatum | 15.000 | 15.000 | 15.000 | 15.000 | 15.000 |
| 15. Water | Q.S. | Q.S. | Q.S. | Q.S. | Q.S. |

| | | | | | |
|---|---|---|---|---|---|
| **(1)** ZPT having an average particle size of 2.5µm, available under the OMADINE trademark from Arch Chemicals, Inc. **(2)** Basic Zinc Carbonate Available from Bruggemann Chemical **(3)** Materials made by reported methods in Lagaly, G.; et al. Inorg. Chem. 1993, 32, 1209-1215 & Morioka, H.; et al. Inorg. Chem. 1999, 38, 4211-4216 **(4)** Polymer JR30M available from Amerchol Corp. | | | | | |

### Cleansing/ Facial Compositions - Proposed Examples 45-54

A suitable method for preparing the anti-microbial cleansing/ facial compositions described in Proposed Examples 45-54 are known to those skilled in the art, and may be prepared by any known or otherwise effective technique, suitable for providing an anti-microbial cleansing/ facial composition provided that the resulting composition provides the excellent anti-microbial benefits described herein. Methods for preparing the anti-microbial cleansing/ facial compositions embodiments of the present invention include conventional formulation and mixing techniques. A method such as that described in U.S. Pat. No. 5,665,364, could be employed.

| **Components** | **Example 45** | **Example 46** | **Example 47** | **Example 48** | **Example 49** |
|---|---|---|---|---|---|
| Cetyl Betaine | 6.667 | 6.667 | 6.667 | 6.667 | 6.667 |
| PPG-15 Stearyl Ether | 4.000 | 4.000 | 4.000 | 4.000 | 4.000 |
| Sodium Lauryl Sulfate | 3.571 | 3.571 | 3.571 | 3.571 | 3.571 |
| Glycerin | 3.000 | 3.000 | 3.000 | 3.000 | 3.000 |
| Stearyl Alcohol | 2.880 | 2.880 | 2.880 | 2.880 | 2.880 |
| Distearyldimonium Chloride | 1.500 | 1.500 | 1.500 | 1.500 | 1.500 |
| Oxidized Polyethylene | 1.000 | 1.000 | 1.000 | 1.000 | 1.000 |
| Zinc Pyrithione (1) | 1.000 | 1.000 | 1.000 | 2.000 | 2.000 |
| Basic Zinc Carbonate (2) | 1.610 | | | | |
| Zinc Hydroxy Sulfate (3) | | 2.000 | | | |
| Zinc Hydroxy Nitrate (3) | | | 1.880 | | |
| Zinc Hydroxy Chloride (3) | | | | 1.630 | |
| Zinc Hydroxy Lauryl Sulfate (3) | | | | | 2.400 |
| Cetyl Alcohol | 0.800 | 0.800 | 0.800 | 0.800 | 0.800 |
| Steareth-21 | 0.500 | 0.500 | 0.500 | 0.500 | 0.500 |
| Behenyl Alcohol | 0.320 | 0.320 | 0.320 | 0.320 | 0.320 |
| PPG-30 | 0.250 | 0.250 | 0.250 | 0.250 | 0.250 |
| Steareth-2 | 0.250 | 0.250 | 0.250 | 0.250 | 0.250 |
| Perfume | 0.200 | 0.200 | 0.200 | 0.200 | 0.200 |
| Citric Acid | As Needed | As Needed | As Needed | As Needed | As Needed |
| Sodium Citrate | As Needed | As Needed | As Needed | As Needed | As Needed |
| Water | Q.S. | Q.S. | Q.S. | Q.S. | Q.S. |

| | | | | | |
|---|---|---|---|---|---|
| **(1)** ZPT having an average particle size of 2.5µm, available under the OMADINE trademark from Arch Chemicals, Inc. **(2)** Basic Zinc Carbonate Available from Bruggemann Chemical **(3)** Materials made by reported methods in Lagaly, G.; et al. Inorg. Chem. 1993, 32, 1209-1215 & Morioka, H.; et al. Inorg. Chem. 1999, 38, 4211-4216 | | | | | |

| Components | **Example 50** | **Example 51** | **Example 52** | **Example 53** | **Example 54** |
|---|---|---|---|---|---|
| Sodium Laureth Sulfate | 8.000 | 8.000 | 8.000 | 8.000 | 8.000 |
| Disodium Cocamphodiacetate | 7.000 | 7.000 | 7.000 | 7.000 | 7.000 |
| PEG-80 Glyceryl Cocoate | 3.500 | 3.500 | 3.500 | 3.500 | 3.500 |
| Sodium Chloride | 2.170 | 2.170 | 2.170 | 2.170 | 2.170 |
| Glycol Distearate | 2.000 | 2.000 | 2.000 | 2.000 | 2.000 |
| Zinc Pyrithione (1) | 1.000 | 1.000 | 1.000 | 2.000 | 2.000 |
| Basic Zinc Carbonate (2) | 1.610 | | | | |
| Zinc Hydroxy Sulfate (3) | | 2.000 | | | |
| Zinc Hydroxy Nitrate (3) | | | 1.880 | | |
| Zinc Hydroxy Chloride (3) | | | | 1.630 | |
| Zinc Hydroxy Lauryl Sulfate (3) | | | | | 2.400 |
| Dimethicone | 0.900 | 0.900 | 0.900 | 0.900 | 0.900 |
| Sodium Trideceth-7 Carboxylate | 0.502 | 0.502 | 0.502 | 0.502 | 0.502 |
| Perfume | 0.320 | 0.320 | 0.320 | 0.320 | 0.320 |
| Citric Acid | 0.276 | 0.276 | 0.276 | 0.276 | 0.276 |
| Quaternium-15 | 0.150 | 0.150 | 0.150 | 0.150 | 0.150 |
| Polyquaterium-10 (11) | 0.150 | 0.150 | 0.150 | 0.150 | 0.150 |
| PEG-30 Glyceryl Cocoate | As Needed | As Needed | As Needed | As Needed | As Needed |
| Water | Q.S. | Q.S. | Q.S. | Q.S. | Q.S. |

| | | | | | |
|---|---|---|---|---|---|
| **(1)** ZPT having an average particle size of 2.5µm, available under the OMADINE trademark from Arch Chemicals, Inc. **(2)** Basic Zinc Carbonate Available from Bruggemann Chemical **(3)** Materials made by reported methods in Lagaly, G.; et al. Inorg. Chem. 1993, 32, 1209-1215 & Morioka, H.; et al. Inorg. Chem. 1999, 38, 4211-4216 | | | | | |

### Hair Conditioning Composition - Proposed Examples 55-78

A suitable method for preparing the anti-microbial hair conditioning compositions described in Proposed Examples 55-78 (below) by conventional formulation and mixing techniques follows:
When included in the composition, polymeric materials such as polypropylene glycol are dispersed in water at room temperature to make a polymer solution, and heated up to above 70°C. Amidoamine and acid, and when present, other cationic surfactants, ester oil of low melting point oil are added in the solution with agitation. Then high melting point fatty compound, and when present, other low melting point oils and benzyl alcohol are also added in the solution with agitation. The mixture thus obtained is cooled down to below 60°C, and the remaining components such as zinc pyrithione, zinc containing material, zinc ionophoric material and silicone compound are added with agitation, and further cooled down to 30°C.

A triblender and/or mill can be used in each step, if necessary to disperse the materials. Alternatively, up to 50% of the acid can be added after cooling below 60°C.

The embodiments disclosed herein have many advantages. For example, they can provide effective anti-microbial, especially anti-dandruff, efficacy, while not deteriorating conditioning benefits such as wet hair feel, spreadability, and rinsability, as well as providing glossiness, and dry combing.

| Components | **Example 55** | **Example 56** | **Example 57** | **Example 58** | **Example 59** | **Example 60** | **Example 61** | **Example 62** | **Example 63** |
|---|---|---|---|---|---|---|---|---|---|
| L-Glutamic Acid | 0.640 | 0.640 | 0.640 | 0.640 | 0.640 | 0.640 | 0.640 | 0.640 | 0.640 |
| Stearamidopropyldimethylamine | 2.000 | 2.000 | 2.000 | 2.000 | 2.000 | 2.000 | 2.000 | 2.000 | 2.000 |
| Behentrimonium Chloride | | | | | | | | | |
| Quaterium-18 | | | | | | | | | |
| Cetyl Alcohol | 2.500 | 2.500 | 2.500 | 2.500 | 2.500 | 2.500 | 2.500 | 2.500 | 2.500 |
| Stearyl Alcohol | 4.500 | 4.500 | 4.500 | 4.500 | 4.500 | 4.500 | 4.500 | 4.500 | 4.500 |
| Cetearyl Alcohol | | | | | | | | | |
| Polysorbate 60 | | | | | | | | | |
| Glyceral Monostearate | | | | | | | | | |
| Oleyl Alcohol | | | | | | | | | |
| Hydroxyethylcellulose | | | | | | | | | |
| Peg 2M (1) | | | | | | | | | |
| Dimethicone (2) | | | | | | | | | |
| Dimethicone (3) | 0.630 | 0.630 | 0.630 | 0.630 | 0.630 | 0.630 | 0.630 | 0.630 | 0.630 |
| Cyclopentasiloxane (3) | 3.570 | 3.570 | 3.570 | 3.570 | 3.570 | 3.570 | 3.570 | 3.570 | 3.570 |
| Benzyl Alcohol | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 |
| Sodium Chloride | 0.010 | 0.010 | 0.010 | 0.010 | 0.010 | 0.010 | 0.010 | 0.010 | 0.010 |
| Zinc Pyrithione (4) | 1.000 | 1.000 | 1.000 | 1.000 | 1.000 | 1.000 | 1.000 | 1.000 | 1.000 |
| Basic Zinc Carbonate (5) | 1.610 | | | | | 0.800 | 0.400 | 2.400 | 3.220 |
| Zinc Hydroxy Sulfate (6) | | 2.000 | | | | | | | |
| Zinc Hydroxy Nitrate (6) | | | 1.880 | | | | | | |
| Zinc Hydroxy Chloride (6) | | | | 1.630 | | | | | |
| Zinc Hydroxy Lauryl Sulfate (6) | | | | | 2.400 | | | | |
| Citric Acid | 0.130 | 0.130 | 0.130 | 0.130 | 0.130 | 0.130 | 0.130 | 0.130 | 0.130 |
| Kathon | | | | | | | | | |
| Perfume | 0.400 | 0.400 | 0.400 | 0.400 | 0.400 | 0.400 | 0.400 | 0.400 | 0.400 |
| Sodium Hydroxide | | | | | | | | | |
| Isopropyl Alcohol | | | | | | | | | |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| **(1)** Polyox WSR N-10 available from Amerchol Corp. **(2)** 10,000cps Dimethicone TSF451-1MA available from GE **(3)** 15/85 Dimethicone/ Cyclomethicone Blend available from GE **(4)** ZPT having an average particle size of 2.5 µm, available under the OMADINE trademark from Arch Chemicals, Inc. **(5)** Basic Zinc Carbonate Available from Bruggemann Chemical **(6)** Materials made by reported methods in Lagaly, G.; et al. Inorg. Chem. 1993, 32, 1209-1215 & Morioka, H.; et al. Inorg. Chem. 1999, 38, 4211-4216 | | | | | | | | | |

| **Components** | **Example 64** | **Example 65** | **Example 66** | **Example 67** | **Example 68** | **Example 69** | **Example 70** | **Example 71** | **Example 72** |
|---|---|---|---|---|---|---|---|---|---|
| L-Glutamic Acid | | | | | | | | | |
| Stearamidopropyldimethylamine | 1.000 | 1.000 | 1.000 | 1.000 | 1.000 | 1.000 | 1.000 | 1.000 | |
| Behentrimonium Chloride | | | | | | | | | 3.380 |
| Quaterium-18 | 0.750 | 0.750 | 0.750 | 0.750 | 0.750 | 0.750 | 0.750 | 0.750 | |
| Cetyl Alcohol | 0.960 | 0.960 | 0.960 | 0.960 | 0.960 | 0.960 | 0.960 | 0.960 | 2.320 |
| Stearyl Alcohol | 0.640 | 0.640 | 0.640 | 0.640 | 0.640 | 0.640 | 0.640 | 0.640 | 4.180 |
| Cetearyl Alcohol | 0.500 | 0.500 | 0.500 | 0.500 | 0.500 | 0.500 | 0.500 | 0.500 | |
| Polysorbate 60 | 0.500 | 0.500 | 0.500 | 0.500 | 0.500 | 0.500 | 0.500 | 0.500 | |
| Glyceral Monostearate | 0.250 | 0.250 | 0.250 | 0.250 | 0.250 | 0.250 | 0.250 | 0.250 | |
| Oleyl Alcohol | 0.250 | 0.250 | 0.250 | 0.250 | 0.250 | 0.250 | 0.250 | 0.250 | |
| Hydroxyethylcellulose | 0.250 | 0.250 | 0.250 | 0.250 | 0.250 | 0.250 | 0.250 | 0.250 | |
| Peg 2M (1) | 0.500 | 0.500 | 0.500 | 0.500 | 0.500 | 0.500 | 0.500 | 0.500 | |
| Dimethicone (2) | | | | | | 0.252 | 0.252 | 0.252 | |
| Dimethicone (3) | 0.630 | 0.630 | 0.630 | 0.630 | 0.630 | | | | 0.630 |
| Cyclopentasiloxane (3) | 3.570 | 3.570 | 3.570 | 3.570 | 3.570 | | | | 3.570 |
| Benzyl Alcohol | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 |
| Sodium Chloride | | | | | | | | | |
| Zinc Pyrithione (4) | 1.000 | 1.000 | 2.000 | 1.000 | 2.000 | 2.000 | 2.000 | 0.500 | 1.000 |
| Basic Zinc Carbonate (5) | 0.800 | | | | | | 1.610 | 1.610 | 0.400 |
| Zinc Hydroxy Sulfate (6) | | 2.000 | 1.000 | | | | | | |
| Zinc Hydroxy Nitrate (6) | | | | 1.880 | | | | | |
| Zinc Hydroxy Chloride (6) | | | | | 1.630 | | | | |
| Zinc Hydroxy Lauryl Sulfate (6) | | | | | | 2.400 | | | |
| Citric Acid | 0.200 | 0.200 | 0.200 | 0.200 | 0.200 | 0.200 | 0.200 | 0.200 | |
| Kathon | | | | | | | | | |
| Perfume | 0.400 | 0.400 | 0.400 | 0.400 | 0.400 | 0.400 | 0.400 | 0.400 | 0.300 |
| Sodium Hydroxide | | | | | | | | | 0.014 |
| Isopropyl Alcohol | | | | | | | | | 0.507 |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| **(1)** Polyox WSR N-10 available from Amerchol Corp. **(2)** 10,000cps Dimethicone TSF451-1MA available from GE **(3)** 15/85 Dimethicone/ Cyclomethicone Blend available from GE **(4)** ZPT having an average particle size of 2.5 µm, available under the OMADINE trademark from Arch Chemicals, Inc. **(5)** Basic Zinc Carbonate Available from Bruggemann Chemical **(6)** Materials made by reported methods in Lagaly, G.; et al. Inorg. Chem. 1993, 32, 1209-1215 & Morioka, H.; et al. Inorg. Chem. 1999, 38, 4211-4216 | | | | | | | | | |

| **Components** | **Example 73** | **Example 74** | **Example 75** | **Example 76** | **Example 77** | **Example 78** |
|---|---|---|---|---|---|---|
| L-Glutamic Acid | | | | | | |
| Stearamidopropyldimethylamine | | | | | | |
| Behentrimonium Chloride | 3.380 | 3.380 | 3.380 | 3.380 | 3.380 | 3.380 |
| Quaterium-18 | | | | | | |
| Cetyl Alcohol | 2.320 | 2.320 | 2.320 | 2.320 | 2.320 | 2.320 |
| Stearyl Alcohol | 4.180 | 4.180 | 4.180 | 4.180 | 4.180 | 4.180 |
| Cetearyl Alcohol | | | | | | |
| Polysorbate 60 | | | | | | |
| Glyceral Monostearate | | | | | | |
| Oleyl Alcohol | | | | | | |
| Hydroxyethylcellulose | | | | | | |
| Peg 2M (1) | | | | | | |
| Dimethicone (2) | | | | | | |
| Dimethicone (3) | 0.630 | 0.630 | 0.630 | 0.630 | 0.630 | 0.630 |
| Cyclopentasiloxane (3) | 3.570 | 3.570 | 3.570 | 3.570 | 3.570 | 3.570 |
| Benzyl Alcohol | 0.400 | 0.400 | 0.400 | 0.400 | 0.400 | 0.400 |
| Methyl Paraben | 0.200 | 0.200 | 0.200 | 0.200 | 0.200 | 0.200 |
| Propyl Paraben | 0.100 | 0.100 | 0.100 | 0.100 | 0.100 | 0.100 |
| Phenoxy Ethanol | 0.300 | 0.300 | 0.300 | 0.300 | 0.300 | 0.300 |
| Sodium Chloride | | | | | | |
| Zinc Pyrithione (4) | 1.000 | 1.000 | 2.000 | 2.000 | 2.000 | 0.500 |
| Basic Zinc Carbonate (5) | | | | | 1.610 | 1.610 |
| Zinc Hydroxy Sulfate (6) | 2.000 | | | | | |
| Zinc Hydroxy Nitrate (6) | | 1.880 | | | | |
| Zinc Hydroxy Chloride (6) | | | 1.630 | | | |
| Zinc Hydroxy Lauryl Sulfate (6) | | | | 2.400 | | |
| Citric Acid | | | | | | |
| Kathon | | | | | | |
| Perfume | 0.300 | 0.300 | 0.300 | 0.300 | 0.300 | 0.300 |
| Sodium Hydroxide | 0.014 | 0.014 | 0.014 | 0.014 | 0.014 | 0.014 |
| Isopropyl Alcohol | 0.507 | 0.507 | 0.507 | 0.507 | 0.507 | 0.507 |
| Water | Q.S. | Q.S. | Q.S. | Q.S. | Q.S. | Q.S. |

| | | | | | | |
|---|---|---|---|---|---|---|
| **(1)** Polyox WSR N-10 available from Amerchol Corp. **(2)** 10,000cps Dimethicone TSF451-1MA available from GE **(3)** 15/85 Dimethicone/ Cyclomethicone Blend available from GE **(4)** ZPT having an average particle size of 2.5 µm, available under the OMADINE trademark from Arch Chemicals, Inc. **(5)** Basic Zinc Carbonate Available from Bruggemann Chemical **(6)** Materials made by reported methods in Lagaly, G.; et al. Inorg. Chem. 1993, 32, 1209-1215 & Morioka, H.; et al. Inorg. Chem. 1999, 38, 4211-4216 | | | | | | |

### Anti-Microbial Leave-In Hair Tonic -Proposed Examples 79-85

A suitable method for preparing the anti-microbial leave-in hair tonic compositions described in Proposed Examples 79-85 (below) follows:
Add most of the formula water; with stirring, add carbomer and mix until fully dispersed. In a separate vessel, add ethanol and then molten PEG-60 hydrogenated castor oil and perfume. Transfer this to main mix tank with agitation. Add other water soluble ingredients, minors, zinc pyrithione, zinc containing materials and/or zinc ionophoric materials. Slowly add styryl silicone and let stir. Add triethanolamine slowly with stirring.

| | **Weight Percent** | | | | | | |
|---|---|---|---|---|---|---|---|
| **Components** | **Example 79** | **Example 80** | **Example 81** | **Example 82** | **Example 83** | **Example 84** | **Example 85** |
| Carbomer | 0.50 | 0.50 | 0.50 | 0.50 | 0.50 | 0.50 | 0.50 |
| Triethanolamine | 0.30 | 0.30 | 0.30 | 0.30 | 0.30 | 0.30 | 0.30 |
| Ethanol | 25.00 | 25.00 | 25.00 | 25.00 | 25.00 | 25.00 | 25.00 |
| Zinc Pyrithione (1) | 0.10 | 0.10 | 0.10 | 0.10 | 0.20 | 0.20 | 0.10 |
| Camphor | 0.05 | 0.05 | 0.05 | 0.05 | 0.05 | 0.05 | 0.05 |
| Menthol | 0.50 | 0.50 | 0.50 | 0.50 | 0.50 | 0.50 | 0.50 |
| Panthenol | 0.05 | 0.05 | 0.05 | 0.05 | 0.05 | 0.05 | 0.05 |
| Pantyl Ethyl Ether | 0.05 | 0.05 | 0.05 | 0.05 | 0.05 | 0.05 | 0.05 |
| Basic Zinc Carbonate (2) | 1.61 | 0.80 | 0.40 | 0.20 | 0.80 | 0.40 | 0.20 |
| Lactic Acid | 0.05 | 0.05 | 0.05 | 0.05 | 0.05 | 0.05 | 0.05 |
| Styryl Silicone | 0.50 | 0.50 | 0.50 | 0.50 | 0.50 | 0.50 | 0.50 |
| Ceteareth-20 | 0.15 | 0.15 | 0.15 | 0.15 | 0.15 | 0.15 | 0.15 |
| PEG-60 Hydrogenated Castor Oil | 0.15 | 0.15 | 0.15 | 0.15 | 0.15 | 0.15 | 0.15 |
| Perfume | 0.50 | 0.50 | 0.50 | 0.50 | 0.50 | 0.50 | 0.50 |
| Water | Q.S. | Q.S. | Q.S. | Q.S. | Q.S. | Q.S. | Q.S. |

| | | | | | | | |
|---|---|---|---|---|---|---|---|
| (1) ZPT having an average particle size of 2.5µm, available under the OMADINE trademark from Arch Chemicals, Inc. (2) Basic Zinc Carbonate Available from Bruggemann Chemical | | | | | | | |

### Anti-microbial Foot Powder - Proposed Examples 86-89

The foot powder composition of Proposed Examples 86-89 is prepared by thoroughly mixing the ingredients in a mixing vessel. The powder may then be ground and/or sifted if necessary.

| | **Weight Percent** | | | |
|---|---|---|---|---|
| **Components** | **Example 86** | **Example 87** | **Example 88** | **Example 89** |
| Talc | 73.25% | 73.25% | 73.25% | 73.7% |
| Calcium Propionate | 15.0 | 15.0 | 15.0 | 15.0 |
| Zinc Propionate | 5.0 | 5.0 | 5.0 | 5.0 |
| Zinc Caprylate | 5.0 | 5.0 | 5.0 | 5.0 |
| Propionic Acid | 0.25 | 0.25 | 0.25 | 0.25 |
| Zinc Sulfate | 0.50 | 0.50 | 0.50 | |
| Zinc Pyrithione (1) | 1.0 | 1.0 | 2.0 | 2.0 |
| Basic Zinc Carbonate (2) | 1.6 | 1.0 | 0.5 | 0.2 |
| | 100.00 | 100.00 | 100.00 | 100.00 |

| | | | | |
|---|---|---|---|---|
| **(1)** ZPT having an average particle size of 2.5 µm, available under the OMADINE trademark from Arch Chemicals, Inc. **(2)** Basic Zinc Carbonate Available from Bruggemann Chemical | | | | |

### Oil-in-Water cream/lotion - Proposed Examples 90-94

The oil-in-water cream/lotion compositions of Proposed Examples 90-94 may be prepared by any known or otherwise effective conventional method.

| | **Weight Percent** | | | | |
|---|---|---|---|---|---|
| **Components** | **Example 90** | **Example 91** | **Example 92** | **Example 93** | **Example 94** |
| Oil Phase | | | | | |
| Mineral oil | 15.0 | 20.0 | 20.0 | 20.0 | 20.0 |
| Polysorbate | 2.0 | 3.0 | 3.0 | 3.0 | 3.0 |
| Aqueous Phase | | | | | |
| Zinc pyrithione (1) | 1.0 | 1.0 | 1.0 | 2.0 | 2.0 |
| Basic Zinc Carbonate (2) | 1.6 | 0.8 | 0.4 | 1.6 | 0.2 |
| Preservative | 0.3 | 0.3 | 0.3 | 0.3 | 0.3 |
| Perfume | 0.2 | 0.2 | 0.2 | 0.2 | 0.2 |
| Water | Q.S. | Q.S. | Q.S. | Q.S. | Q.S. |

| | | | | | |
|---|---|---|---|---|---|
| **(1)** ZPT having an average particle size of 2.5µm, available under the OMADINE trademark from Arch Chemicals, Inc. **(2)** Basic Zinc Carbonate Available from Bruggemann Chemical | | | | | |

### 10. Other Ingredients

The present invention may, in some embodiments, further comprise additional optional components known or otherwise effective for use in hair care or personal care products. The concentration of such optional ingredients generally ranges from zero to 25%, more typically from 0.05% to 20%, even more typically from 0.1% to 15%, by weight of the composition. Such optional components should also be physically and chemically compatible with the essential components described herein, and should not otherwise unduly impair product stability, aesthetics or performance.

Examples of optional components for use in the present invention include anti-static agents, foam boosters, anti-dandruff agents in addition to the anti-dandruff agents described above, viscosity adjusting agents and thickeners, suspension materials (e.g. EGDS, thixins), pH adjusting agents (e.g. sodium citrate, citric acid, succinic acid, sodium succinate, sodium maleate, sodium glycolate, malic acid, glycolic acid, hydrochloric acid, sulfuric acid, sodium bicarbonate, sodium hydroxide, and sodium carbonate), preservatives (e.g. DMDM hydantoin), anti-microbial agents (e.g. triclosan or triclocarbon), dyes, organic solvents or diluents, pearlescent aids, perfumes, fatty alcohols, proteins, skin active agents, sunscreens, vitamins (such as retinoids including retinyl propionate, vitamin E such as tocopherol acetate, panthenol, and vitamin B3 compounds including niacinamide), emulsifiers,volatile carriers, select stability actives, styling polymers, organic styling polymers, silicone-grafted styling polymers, cationic spreading agents, pediculocides, foam boosters, viscosity modifiers and thickeners, polyalkylene glycols and combinations thereof.

Optional anti-static agents such as water-insoluble cationic surfactants may be used, typically in concentrations ranging from 0.1% to 5%, by weight of the composition. Such anti-static agents should not unduly interfere with the in-use performance and end-benefits of the anti-microbial composition; particularly, the anti-static agent should not interfere with the anionic surfactant. A specific example of a suitable anti-static agents is tricetyl methyl ammonium chloride.

Optional foam boosters for use in the present invention described herein include fatty ester (e.g. C₈-C₂₂) mono- and di (C₁-C₅, especially C₁-C₃) alkanol amides. Specific examples of such foam boosters include coconut monoethanolamide, coconut diethanolamide, and mixtures thereof.

Optional viscosity modifiers and thickeners may be used, typically in amounts effective for the anti-microbial compositions of the present invention to generally have an overall viscosity from 1,000 csk to 20,000 csk, preferably from 3,000 csk to 10,000 csk. Specific examples of such viscosity modifiers and thickeners include: sodium chloride, sodium sulfate, and mixtures thereof.

### M. Other Preferred Embodiments:

Other preferred embodiments of the present invention include the following:
An embodiment of the present invention relates to a composition comprising an antimicrobially effective amount of pyrithione and zinc-containing layered material in an amount sufficient to enhance the efficacy of component with the proviso that component (b) is present in a weight ratio of from 5:100 to 10:1 based upon the amount of component (a) present in
said composition. Preferably component (a) is zinc pyrithione and component (b) is basic zinc carbonate.

In another embodiment of the present invention, a process for preparing a basic zinc carbonate-containing personal care composition selected from the group consisting of shampoo, soap, skin care medicament, and combinations thereof is disclosed, said process comprising reacting, in a personal care composition comprising pyrithione or a polyvalent metal salt of a pyrithione, a carbonate or bicarbonate salt that is soluble in the personal care composition with a zinc compound that is soluble or insoluble in the personal care composition, said zinc compound being selected from the group consisting of zinc salts of organic acids, zinc salts of inorganic acids, zinc hydroxide, zinc oxide, and combinations thereof, thereby causing in-situ formation of the carbonate salt with the zinc salt to form said basic zinc carbonate in said basic zinc carbonate-containing personal care composition. Preferably, said zinc compound is zinc hydroxide and said carbonate salt is sodium carbonate, and wherein said zinc hydroxide, and said zinc hydroxide is reacted with said sodium carbonate in a molar ratio within a range of between 1:10 and 10:1.

In a further embodiment of the present invention, according to the process as described above, the pyrithione or a polyvalent metal salt of a pyrithione and the basic zinc carbonate are simultaneously or step wise generated in situ in the personal care composition.

In another embodiment of the present invention, a personal care composition selected from the group consisting of shampoo, soap, skin care medicament, and combinations thereof is discloses, said personal care composition comprising water, alcohol, or a combination thereof, pyrithione or a polyvalent metal salt of a pyrithione, and as an augmentation agent for enhancing the antimicrobial efficacy of said pyrithione or polyvalent metal salt of pyrithione, particles or a layered film of an in-situ reaction product of a zinc compound selected from the group consisting of zinc salts of organic acids, zinc salts of inorganic acids, zinc hydroxide, zinc oxide, and combinations thereof, said zinc compound being soluble in said water or alcohol, with a carbonate salt other than basic zinc carbonate that is soluble in said water or alcohol.

Typically, augmentation factors greater than 50 are not envisioned. Typically an augmentation factor is envisioned to be less than 50, preferably less than 25, more preferably less than 10.

In a further embodiment of the present invention, a process for preparing a basic zinc carbonate-containing personal care composition selected from the group consisting of shampoo, soap, skin care medicament, and combinations thereof is disclosed, said process comprising reacting, in a personal care composition, a carbonate or bicarbonate salt that is soluble in the personal care composition with a zinc compound that is soluble or insoluble in the personal care composition, said zinc compound being selected from the group consisting of zinc salts of organic acids, zinc salts of inorganic acids, zinc hydroxide, zinc oxide, and combinations thereof, thereby causing in-situ formation of the carbonate salt with the zinc salt to form said basic zinc carbonate in said basic zinc carbonate-containing personal care composition.

## Claims

1. A composition comprising:
a) an effective amount of zinc pyrithione comprised from 0.01% to 5%, based upon the total weight of the composition ;
b) an effective amount of a zinc-containing layered material comprised from 0.001% to 10%, based upon the total weight of the composition,
wherein the zinc-containing layered material is selected from the group consisting of basic zinc carbonate, zinc carbonate hydroxide, hydrozincite, zinc copper carbonate hydroxide, aurichalcite, copper zinc carbonate hydroxide, rosasite, phyllosilicate containing zinc ions, layered double hydroxide, hydroxy double salts and mixtures thereof, preferably wherein the zinc-containing layered material is selected from the group consisting of zinc carbonate hydroxide, hydrozincite, basic zinc carbonate and mixtures thereof, more preferably wherein the zinc-containing layered material is hydrozincite or basic zinc carbonate, most preferably wherein the zinc containing layered material is basic zinc carbonate.

2. A composition according to Claim 1 wherein a weight ratio of zinc-containing layered material to zinc pyrithione is from 5:100 to 10:1.

3. A composition according to Claim 1 wherein the zinc-containing layered material is present from 0.01% to 7%, preferably wherein the zinc-containing layered material is present from 0.1% to 5%, based upon the total weight of the composition.

4. A composition according to Claim 1 wherein the zinc pyrithione is present from 0.1% to 2%, based upon the total weight of the composition.

5. A composition according to Claim 1 for treating microbial infections.

6. A composition according to Claim 1 for treating fungal infections.

7. A composition according to Claim 1 for treating dandruff.

## Patentansprüche

1. Zusammensetzung, umfassend:
a) eine wirksame Menge an Zink-Pyrithion, enthalten in der Menge von 0,01 % bis 5 %, bezogen auf das Gesamtgewicht der Zusammensetzung;
b) eine wirksame Menge eines zinkhaltigen geschichteten Materials, enthalten in der Menge von 0,001 % bis 10 %, bezogen auf das Gesamtgewicht der Zusammensetzung,
wobei das zinkhaltige, geschichtete Material ausgewählt ist aus der Gruppe bestehend aus basischem Zinkcarbonat, Zinkcarbonathydroxid, Hydrozinkit, Zinkkupfercarbonathydroxid, Aurichalzit, Kupferzinkcarbonathydroxid, Rosasit, phyllosilicathaltigen Zinkionen, geschichtetem Doppelhydroxid, Hydroxydoppelsalzen und Mischungen davon, wobei das zinkhaltige, geschichtete Material vorzugsweise ausgewählt ist aus der Gruppe bestehend aus Zinkcarbonathydroxid, Hydrozinkit, basischem Zinkcarbonat und Mischungen davon, wobei das zinkhaltige, geschichtete Material mehr bevorzugt Hydrozinkit oder basisches Zinkcarbonat ist, wobei das zinkhaltige, geschichtete Material am meisten bevorzugt basisches Zinkcarbonat ist.

2. Zusammensetzung nach Anspruch 1, wobei ein Gewichtsverhältnis des zinkhaltigen, geschichteten Materials zu Zink-Pyrithion von 5:100 bis 10:1 beträgt.

3. Zusammensetzung nach Anspruch 1, wobei das zinkhaltige, geschichtete Material in einer Menge von 0,01 % bis 7 % vorhanden ist, wobei vorzugsweise das zinkhaltige, geschichtete Material in einer Menge von 0,1 % bis 5 %, bezogen auf das Gesamtgewicht der Zusammensetzung, vorhanden ist.

4. Zusammensetzung nach Anspruch 1, wobei das Zink-Pyrithion in einer Menge von 0,1 % bis 2 %, bezogen auf das Gesamtgewicht der Zusammensetzung, vorhanden ist.

5. Zusammensetzung nach Anspruch 1 zur Behandlung von mikrobiellen Infektionen.

6. Zusammensetzung nach Anspruch 1 zur Behandlung von Pilzinfektionen.

7. Zusammensetzung nach Anspruch 1 zur Behandlung von Schuppen.

## Revendications

1. Composition comprenant :
a) une quantité efficace de pyrithione de zinc comprise de 0,01 % à 5 %, sur la base du poids total de la composition ;
b) une quantité efficace d'un matériau en couches contenant du zinc compris de 0,001 % à 10 %, sur la base du poids total de la composition,
dans laquelle le matériau en couches contenant du zinc est choisi dans le groupe constitué de carbonate de zinc basique, carbonate hydroxyde de zinc, hydrozincite, carbonate hydroxyde de zinc cuivre, aurichalcite, carbonate hydroxyde de cuivre et de zinc, rosasite, phyllosilicate contenant des ions zinc, hydroxyde double feuilleté, sels doubles hydroxy et leurs mélanges, de préférence dans laquelle le matériau en couches contenant du zinc est choisi dans le groupe constitué de carbonate hydroxyde de zinc, hydrozincite, carbonate de zinc basique et leurs mélanges, plus préférablement dans lequel le matériau en couches contenant du zinc est de l'hydrozincite ou du carbonate de zinc basique, le plus préférablement dans laquelle le matériau en couches contenant du zinc est du carbonate de zinc basique.

2. Composition selon la revendication 1, dans laquelle un rapport pondéral du matériau en couches contenant du zinc à la pyrithione de zinc va de 5:100 à 10:1.

3. Composition selon la revendication 1, dans laquelle le matériau en couches contenant du zinc est présent à raison de 0,01 % à 7 %, de préférence dans laquelle le matériau en couches contenant du zinc est présent à raison de 0,1 % à 5 %, sur la base du poids total de la composition.

4. Composition selon la revendication 1, dans laquelle la pyrithione de zinc est présente à raison de 0,1 % à 2 %, sur la base du poids total de la composition.

5. Composition selon la revendication 1 pour traiter des infections microbiennes.

6. Composition selon la revendication 1 pour traiter des infections fongiques.

7. Composition selon la revendication 1 pour traiter des pellicules.
